# EUROPÄISCHE PATENTANMELDUNG

(11) **EP 2 420 286 A1**
(43) Veröffentlichungstag der Anmeldung: **22.02.2012**
(21) Anmeldenummer: 10173187.5
(22) Anmeldetag: 18.08.2010
(51) Int. Cl.: A61M 39/14, B29C 65/20

(54) **Verfahren und Vorrichtung zum sterilen Verbinden von Schläuchen**

(71) Anmelder: Fresenius Kabi Deutschland GmbH, 61352 Bad Homburg (DE)
(72) Erfinder: Brückner, Thomas, 63776, Mömbris (DE); Schwalm, Michael, 36304, Alsfeld (DE)
(74) Vertreter: Richly, Erik

(57) **Zusammenfassung**

Die Erfindung betrifft ein Verfahren zum sterilen Verbinden von Schläuchen, mit den Schritten:
a) Bereitstellen eines ersten und eines zweiten Schlauchs (1a, 1 b);
b) Erhitzen jeweils eines Trennbereiches des ersten und des zweiten Schlauchs (1a, 1 b) unter Verwendung von Erhitzungsmitteln;
c) mechanisches Auftrennen der beiden Schläuche (1a, 1 b) durch Ausüben einer Zug- und/oder Scherkraft auf die Schläuche, so dass die beiden Schläuche (1a, 1 b) jeweils im erhitzten Trennbereich in einen ersten und einen zweiten Schlauchabschnitt (100a, 100b, 101 a, 101 b) aufgetrennt werden; und
d) nach dem Auftrennen der Schläuche (1a, 1 b) Herstellen eines mechanischen Kontaktes des durch das Auftrennen des ersten Schlauchs gebildeten Endes (1001 a) des ersten Schlauchabschnitts (1 00a) des ersten Schlauchs (1 a) mit dem durch das Auftrennen des zweiten Schlauchs (1 b) gebildeten Ende (1001 b) des ersten Schlauchabschnitts (100b) des zweiten Schlauchs (1b), wobei
e) das Herstellen des mechanischen Kontaktes so erfolgt, dass die Enden (1001 a, 1001 b) der Schlauchabschnitte (100a, 100b) in Folge des Erhitzens gemäß Schritt b) noch eine solche Temperatur aufweisen, wenn sie in mechanischen Kontakt miteinander kommen, dass sie ohne zusätzliches Erhitzen nach dem Auftrennen eine stoffschlüssige Verbindung miteinander eingehen.

Die Erfindung betrifft auch eine Vorrichtung zum sterilen Verbinden von Schläuchen.

## Beschreibung

Die Erfindung betrifft ein Verfahren zum Verbinden von Schläuchen gemäß Anspruch 1 sowie eine Vorrichtung zum Verbinden von Schläuchen gemäß Anspruch 7.

Insbesondere im medizinischen Bereich besteht die Anforderung, Schläuche, die z. B. zum Transport von Blut oder Infusionsflüssigkeiten dienen, steril miteinander verbinden zu können. Bei einem aus der EP 1 555 111 A1 bekannten Verfahren zum sterilen Verbinden von Schläuchen werden beispielsweise zwei Schläuche aus einem thermoplastischen Material zunächst jeweils mittels einer erhitzten Klinge durchtrennt und jeweils zwei Hauptabschnitte der durchtrennten Schläuche dadurch miteinander verbunden, dass sie entlang der erhitzten Klinge aufeinander zu bewegt und nach Entfernen der Klinge aufeinandergedrückt werden. Die Klinge muss nach Verwendung als Sondermüll entsorgt werden.

Das von der vorliegenden Erfindung zu lösende Problem besteht darin, ein Verfahren bereitzustellen, das ein möglichst schnelles Verbinden von Schläuchen ermöglicht und möglichst wenig Abfall erzeugt. Der vorliegenden Erfindung liegt zudem das Problem zugrunde, eine entsprechende Vorrichtung zum Verbinden von Schläuchen zu schaffen.

Die obigen Probleme werden durch das Verfahren mit den Merkmalen gemäß Anspruch 1 sowie durch die Vorrichtung mit den Merkmalen gemäß Anspruch 7 gelöst. Weiterbildungen der Erfindung sind in den abhängigen Ansprüchen angegeben.

Danach wird ein Verfahren zum sterilen Verbinden von Schläuchen bereitgestellt, mit den Schritten:
a) Bereitstellen eines ersten und eines zweiten Schlauchs;
b) Erhitzen jeweils eines Trennbereiches des ersten und des zweiten Schlauchs unter Verwendung von Erhitzungsmitteln;
c) mechanisches Auftrennen der beiden Schläuche durch Ausüben einer Zug- und/oder Scherkraft auf die Schläuche, so dass die beiden Schläuche jeweils im erhitzten Trennbereich in einen ersten und einen zweiten Schlauchabschnitt aufgetrennt werden; und
d) nach dem Auftrennen der Schläuche Herstellen eines mechanischen Kontaktes des durch das Auftrennen des ersten Schlauchs gebildeten Endes des ersten Schlauchabschnitts des ersten Schlauchs mit dem durch das Auftrennen des zweiten Schlauchs gebildeten Ende des ersten Schlauchabschnitts des zweiten Schlauchs, wobei
e) das Herstellen des mechanischen Kontaktes so erfolgt, dass die Enden der Schlauchabschnitte in Folge des Erhitzens gemäß Schritt b) noch eine solche Temperatur aufweisen, wenn sie in mechanischen Kontakt miteinander kommen, dass sie ohne zusätzliches Erhitzen nach dem Auftrennen eine stoffschlüssige Verbindung miteinander eingehen.

Gemäß dem erfindungsgemäßen Verfahren wird somit zum Auftrennen der beiden Schläuche kein Schneidwerkzeug verwendet, sondern das Auftrennen erfolgt dadurch, dass ein Bereich (Trennbereich) des jeweiligen Schlauches erhitzt und eine Zug- und/oder Scherkraft in den durch die Erhitzung erweichten Trennbereich eingeleitet wird. Zum Ausüben einer Zugkraft werden die Schläuche insbesondere jeweils an einem Ende gehalten und eine Kraft entlang einer Längsachse, entlang derer die Schläuche zumindest im Trennbereich verlaufen, erzeugt. Gleichzeitig oder alternativ kann auf die Schläuche jeweils auch eine (insbesondere in dem jeweiligen Trennbereich wirkende) Scherkraft ausgeübt werden, wobei z.B. eine Kraft erzeugt wird, die senkrecht oder schräg zur Längsachse der Schläuche im Trennbereich erzeugt wird.

Beispielsweise wird die Zug- und/oder Scherkraft zum Auftrennen der Schläuche durch eine Rotation und/oder eine Translation eines Teiles des jeweiligen Schlauches erzeugt. Möglichkeiten zur Erzeugung einer Zug- und/oder Scherkraft, die zum Auftrennen der Schläuche im erhitzten Trennbereich führt, werden weiter unten im Einzelnen erläutert.

Nach dem erfindungsgemäßen Verfahren erfolgt vorzugsweise zusätzlich zu dem Erhitzen gemäß Schritt b) kein gesondertes Erhitzen der Enden der ersten Schlauchabschnitte, bevor sie miteinander in Kontakt kommen. Beispielsweise weisen die Enden der Schlauchabschnitte unmittelbar nach dem Auftrennen der Schläuche eine Höchsttemperatur auf und kühlen, bis sie miteinander in Kontakt kommen, auf eine niedrigere Temperatur ab. Diese niedrigere Temperatur ist allerdings noch so hoch, dass sich die beiden Enden ohne weitere Wärmeeinwirkung stoffschlüssig miteinander verbinden. Insbesondere erfolgt das Herstellen des mechanischen Kontaktes zwischen den Enden der ersten Schlauchabschnitte so schnell, dass sie, wenn sie in mechanischen Kontakt miteinander kommen, jeweils noch eine Temperatur aufweisen, die größer als die Schmelztemperatur des Materials des jeweiligen Schlauchabschnitts ist. Beispielsweise wird der Trennbereich der Schläuche erhitzt (z.B. auf über 200°C), bis das (thermoplastische) Schlauchmaterial den thermoplastischen Zustand erreicht. Die Temperatur der Enden der ersten Schlauchabschnitte nach dem Auftrennen der Schläuche bis zu ihrem Verbinden liegt z.B. oberhalb von 100°C.

Die durch das Auftrennen der Schläuche entstandenen Enden der ersten Schlauchabschnitte können aufgrund ihrer anhaltend hohen Temperatur versiegeln, so dass insbesondere ein steriler Verschluss der ersten Schlauchabschnitte entsteht. Dadurch, dass die Enden der ersten Schlauchabschnitte bis zum Verbinden ihrer Enden miteinander eine Temperatur oberhalb des Schmelzpunktes des Schlauchmaterials aufweisen, ist zudem sichergestellt, dass diese Versiegelung bis zum Verbinden der ersten Schlauchabschnitte besteht.

Die beiden Schläuche sind, wie oben bereits erwähnt, insbesondere aus einem thermoplastischen Material (z. B. aus PVC oder einem anderen Kunststoff) gebildet, wobei sich die beiden Enden der ersten Schlauchabschnitte beispielsweise jeweils noch in ihrem thermoplastischen Zustand befinden, wenn sie in Kontakt miteinander kommen. Es ist allerdings nicht zwingend notwendig, dass die beiden Schläuche aus dem gleichen Material bestehen, sondern es können mit dem erfindungsgemäßen Verfahren selbstverständlich auch Schläuche aus unterschiedlichen Materialien, z.B. aus unterschiedlichen thermoplastischen Kunststoffen, miteinander verbunden werden.

Gemäß einer Ausgestaltung der Erfindung beträgt der zeitliche Abstand zwischen dem Auftrennen des ersten und des zweiten Schlauches und dem Zeitpunkt des Vorliegens des mechanischen Kontaktes zwischen den Enden der ersten Schlauchabschnitte höchstens 0,1 1 s, insbesondere höchstens 0,05 s. Der "Zeitpunkt des Vorliegens des mechanischen Kontaktes" ist insbesondere der Zeitpunkt, zu dem eine erste Berührung des Endes des ersten Schlauchabschnitts des ersten Schlauches mit dem Ende des ersten Schlauchabschnitts des zweiten Schlauches auftritt. Insbesondere weisen die miteinander zu verbinden Enden jeweils eine Stirnseite auf, die zum Herstellen des mechanischen Kontaktes gegeneinander gedrückt werden, so dass als "Zeitpunkt des Vorliegens des mechanischen Kontaktes" auch der Zeitpunkt der ersten Berührung der beiden Stirnseiten angesehen werden kann.

Es wird darauf hingewiesen, dass die Erfindung natürlich nicht auf einen bestimmten zeitlichen Abstand zwischen dem Auftrennen der Schläuche und dem Verbinden der ersten Schlauchabschnitte festgelegt ist. Denkbar sind auch deutlich längere zeitliche Abstände als die oben angegebenen, sofern das Herstellen des mechanischen Kontaktes ausreichend schnell erfolgt, um ein stoffschlüssiges Verbinden der Enden der ersten Schlauchabschnitte ohne einen weiteren Erhitzungsschritt zu ermöglichen. Beispielsweise kann der betrachtete zeitliche Abstand deutlich größer als 0,1 s sein, z.B. bis zu einer Sekunde betragen.

Das Herstellen des mechanischen Kontaktes zwischen den Enden der beiden ersten Schlauchabschnitte umfasst insbesondere ein Wegbewegen des jeweiligen ersten Schlauchabschnittes (z.B. in Form eines Hauptschlauchabschnitts, der eine größere Länge als der zweite Schlauchabschnitt aufweist) von dem jeweiligen zweiten Schlauchabschnitt (z.B. in Form eines Endes des ursprünglichen Schlauches) und ein Aufeinanderzubewegen der beiden ersten Schlauchabschnitte, ein Ausrichten ihrer Enden entlang einer gemeinsamen Achse und ein Gegeneinanderdrücken der beiden Enden (insbesondere ihrer Stirnseiten).

Beispielsweise umfasst das Aufeinanderzubewegen der beiden ersten Schlauchabschnitte eine Rotation zumindest eines Teils des ersten Schlauchabschnittes des ersten Schlauches und/oder eine Rotation zumindest eines Teils des ersten Schlauchabschnittes des zweiten Schlauches. Hier sind die beiden ersten Schlauchabschnitte (des ersten und des zweiten Schlauches) z.B. jeweils an einer Aufnahmevorrichtung so gelagert, dass sie nach Auftrennen des ersten bzw. des zweiten Schlauches durch Rotation aufeinander zu bewegt werden können. Auf derartige rotierbare Aufnahmevorrichtungen wird weiter unten noch näher eingegangen.

Insbesondere erfolgt die Rotation des ersten Schlauchabschnitts des ersten Schlauches und/oder des ersten Schlauchabschnitts des zweiten Schlauches um eine Rotationsachse, die zumindest näherungsweise senkrecht zu einer Ebene verläuft, entlang derer sich der erste Schlauchabschnitt zumindest teilweise erstreckt. Beispielsweise ist der erste Schlauchabschnitt des ersten Schlauches so gelagert, dass sich sein Ende (das mit dem Ende des ersten Schlauchbereichs des zweiten Schlauches verbunden werden soll) zumindest näherungsweise geradlinig (d.h. entlang einer Längsachse) erstreckt, wobei die Rotationsachse dann senkrecht zu diesem geradlinig verlaufenden Ende orientiert ist. Somit ist unter der Formulierung, dass sich der Schlauchabschnitt "entlang einer Ebene" erstreckt, nicht notwendigerweise gemeint, dass er gekrümmt oder mit zueinander abgewinkelten Abschnitten verläuft. Es ist auch möglich, dass er sich zumindest zum Teil im Wesentlichen geradlinig erstreckt.

Beispielsweise wird eine Vorrichtung zum Ausführen des oben beschriebenen Verfahrens verwendet, die Folgendes umfasst:
- eine erste Aufnahmevorrichtung zur Aufnahme eines ersten Schlauches;
- eine zweite Aufnahmevorrichtung zur Aufnahme eines zweiten Schlauches;
- Erhitzungsmittel zum Erhitzen eines Trennbereiches des ersten und des zweiten Schlauchs;
- Mittel zum Erzeugen einer Zug- und/oder Scherkraft auf die Schläuche, um die beiden Schläuche jeweils im erhitzten Trennbereich in einen ersten und einen zweiten Schlauchabschnitt aufzutrennen;
- Kontakterzeugungsmittel zum Herstellen eines mechanischen Kontaktes des durch das Auftrennen des ersten Schlauchs gebildeten Endes des ersten Schlauchabschnitts des ersten Schlauchs mit dem durch das Auftrennen des zweiten Schlauchs gebildeten Ende des ersten Schlauchabschnitts des zweiten Schlauchs, wobei
- die Kontakterzeugungsmittel ausgebildet sind, die Enden der Schlauchabschnitte nach dem Auftrennen der Schläuche so (schnell) in mechanischen Kontakt miteinander zu bringen, dass sie infolge des Erhitzens unter Verwendung der Erhitzungsmittel noch eine solche Temperatur aufweisen, wenn sie in mechanischen Kontakt miteinander kommen, dass sie ohne erneutes Erhitzen eine stoffschlüssige Verbindung miteinander eingehen.

Die Erfindung betrifft auch eine Vorrichtung zum sterilen Verbinden von Schläuchen, insbesondere zum Durchführen eines wie oben beschriebenen Verfahrens, mit
- einer ersten Aufnahmevorrichtung zur Aufnahme eines ersten Schlauches;
- einer zweiten Aufnahmevorrichtung zur Aufnahme eines zweiten Schlauches;
- Erhitzungsmitteln zum Erhitzen eines Trennbereiches eines an der ersten Aufnahmevorrichtung angeordneten ersten Schlauchs und eines Trennbereichs eines an der zweiten Aufnahmevorrichtung angeordneten zweiten Schlauchs; und
- Auftrennmitteln zum Auftrennen des ersten und des zweiten Schlauchs in dem erhitzten Trennbereich jeweils in einen ersten und einen zweiten Schlauchabschnitt, wobei
- die erste und die zweite Aufnahmevorrichtung jeweils ausgebildet sind, den ersten Schlauchabschnitt jeweils so zu halten, dass er sich zumindest teilweise entlang einer Ebene erstreckt, und zumindest eine der beiden Aufnahmenvorrichtungen um eine Rotationsachse rotierbar gelagert ist, die zumindest näherungsweise senkrecht zu der Ebene verläuft, und wobei
- die Vorrichtung so ausgebildet ist, dass nach Auftrennen des ersten und des zweiten Schlauches ein Ende des ersten Schlauchabschnitts des ersten Schlauches unter Rotation der ersten und/oder der zweiten Aufnahmevorrichtung um die Rotationsachse in mechanischen Kontakt mit einem Ende des ersten Schlauchabschnitts des zweiten Schlauches gebracht werden kann.

Insbesondere erfolgt das Herstellen des Kontaktes zwischen den Enden der ersten Schlauchabschnitte (und insbesondere die Rotation der ersten und/oder der zweiten Aufnahmevorrichtung) so schnell, dass die Enden in Folge des Erhitzens unter Verwendung der Erhitzungsmittel auch nach dem Auftrennen der Schläuche (z.B. auch nach einem Abschalten der Erhitzungsmittel)noch eine solche Temperatur aufweisen, wenn sie in mechanischen Kontakt miteinander kommen, dass sie ohne erneutes Erhitzen eine stoffschlüssige Verbindung miteinander eingehen, wie weiter oben in Bezug auf das erfindungsgemäße Verfahren beschrieben.

Eine derartige Vorrichtung benötigt für das Auftrennen der Schläuche und das Verbinden der ersten Schlauchabschnitte nur eine einzige Wärmequelle (Erhitzungsmittel), da ein gesondertes, nochmaliges Erhitzen der Schlauchabschnitte vor ihrem Verbinden entfällt. Dies ermöglicht eine möglichst kompakte und möglichst kostengünstig herstellbare Vorrichtung. Die mögliche kompakte Bauform wiederum kann geringe Verfahrwege der ersten Schlauchabschnitte, die beim Herstellen des mechanischen Kontaktes ihrer Enden zurückgelegt werden müssen, ermöglichen. Dies kann z.B. Positionierprobleme reduzieren oder beseitigen und ermöglicht insbesondere kurze "Dockzeiten", d.h. das Trennen der Schläuche und Verbinden der ersten Schlauchabschnitte erfolgt in relativ kurzer Zeit (z.B. in weniger als 10 s, z.B. in etwa 6 s). Zudem können auch kurze Schläuche (die z.B. eine Länge von höchstens 3 cm aufweisen) miteinander verbunden werden.

Dadurch, dass nur eine Heizquelle vorgesehen werden muss, ist es darüber hinaus z.B. möglich, eine besonders einfach montierbare und möglichst wirksame Abdeckung vorzusehen, die die erfindungsgemäße Vorrichtung gegen das Eindringen von Feuchtigkeit schützt. Darüber hinaus erfolgt das Verfahren der ersten Schlauchabschnitte zum Inkontaktbringen ihrer Enden im Wesentlichen über eine Rotationsbewegung der Aufnahmevorrichtungen. Eine solche Rotationsbewegung kann durch eine relativ einfache und robuste Mechanik erzeugt werden, so dass z.B. eine möglichst prozesssichere und langzeitstabile Vorrichtung realisiert werden kann. Zum Antreiben der Aufnahmevorrichtungen dient insbesondere ein Elektromotor. Beispielsweise wird jeweils ein Gleichstrommotor verwendet, der insbesondere einen Encoder aufweist, um die Stellung des Motors und damit der jeweiligen Aufnahmevorrichtung ermitteln zu können.

Beispielsweise ist der erste Schlauch mit einem ersten Behälter (z.B. Blutbeutel) und der zweite Schlauch mit einem zweiten Behälter, der z.B. einen Blutzusatz enthält, verbunden. Das erfindungsgemäße Verfahren kann hier verwendet werden, um die beiden Behälter miteinander zu verbinden, indem von beiden Schläuchen ein "freies" Schlauchenden (die "zweiten Schlauchabschnitte") abgetrennt und die verbleibenden Hauptschlauchabschnitte (die "ersten Schlauchabschnitte") miteinander verbunden ("gedockt") werden. Eine andere Anwendung besteht z.B. in dem Verbinden eines blutführenden Schlauches mit einem Leukozytenfilter.

Es wird darauf hingewiesen, dass die Elemente, die bereits weiter oben in Bezug auf das erfindungsgemäße Verfahren beschrieben wurden, natürlich auch in dieser Vorrichtung verwirklicht sein können. Beispielsweise kann die Zeitspanne, die vom Auftrennen bis zum Inkontaktbringen der Enden der ersten Schlauchabschnitte vergeht, weniger als 0,1 s betragen.

Des Weiteren wird, wie oben bereits erwähnt, darauf hingewiesen, dass die Formulierung, wonach die ersten Schlauchabschnitte "entlang einer Ebene" verlaufen, natürlich auch den Fall erfasst, dass die ersten Schlauchabschnitte an den Aufnahmevorrichtungen so gehalten werden, dass sie sich (zumindest zum Teil) im Wesentlichen geradlinig (d.h. entlang einer Längsachse) erstrecken.

Beispielsweise ist jeder der Aufnahmevorrichtungen mindestens eine Klemmvorrichtung zum Klemmen des an der Aufnahmevorrichtung angeordneten Schlauches zugeordnet, insbesondere, um den Schlauch in Vorbereitung des Auftrennens zusammenzudrücken und/oder ihn zu halten (z.B. an einem Träger, an dem die Klemmvorrichtung festgelegt ist). Insbesondere ist diese Klemmvorrichtung so angeordnet, dass sie sich bei Rotation der Aufnahmevorrichtung nicht mitbewegt. Es ist denkbar, dass die Aufnahmevorrichtungen und die jeweilige Klemmvorrichtung auf einem gemeinsamen Träger angeordnet sind, wobei jedoch nur die Aufnahmevorrichtungen (bzw. zumindest eine der Aufnahmevorrichtungen) relativ zu dem Träger rotierbar sind, nicht jedoch die Klemmvorrichtungen.

Die Klemmvorrichtungen können jeweils zwei Klemmbacken umfassen, zwischen denen der erste bzw. der zweite Schlauch hindurchgeführt wird, und die jeweils aufeinander zu bewegt werden können, um den Schlauch zwischen den Klemmbacken zu klemmen. Beispielsweise werden die beiden Klemmbacken (oder auch nur eine der Klemmbacken) mittels eines Elektromotors rotiert, der z.B. jeweils auch einen Encoder aufweisen kann, um die Stellung der Klemmbacken ermitteln zu können.

Gemäß einer Ausgestaltung der erfindungsgemäßen Vorrichtung umfassen die Erhitzungsmittel Hochfrequenzspannungserzeugungsmittel, die jeweils mit zumindest einer der beiden Klemmvorrichtungen elektrisch verbunden und ausgebildet sind, eine Hochfrequenzspannung in die jeweilige Klemmvorrichtung einzuleiten, um im Bereich der Klemmvorrichtung ein elektrisches Hochfrequenzfeld zu erzeugen. Dieses Hochfrequenzfeld erhitzt (insbesondere über Ankopplung an Dipole des Schlauchmaterials) einen Bereich (den Trennbereich) des mittels der Klemmvorrichtung geklemmten Schlauches.

Beispielsweise weist auch hier die Klemmvorrichtung jeweils zwei Klemmbacken auf, zwischen denen der Schlauch geklemmt werden kann, wobei zumindest in eine der beiden Klemmbacken die Hochfrequenzspannung eingespeist wird. Insbesondere ist die Klemmbacke, in die die Hochfrequenzspannung eingespeist wird, aus Metall (z. B. aus Messing) ausgebildet. Mit anderen Worten bildet zumindest eine der Klemmbacken eine HF-Elektrode, die z.B. anders als konventionelle HF-Elektroden eine plane Seite aufweist, mit der sie in Klemmstellung an dem Schlauch anliegt.

Die Temperatur der Schläuche im Trennbereich und die Größe des erhitzten Schlauchbereichs kann z.B. über die Dauer und/oder die Intensität des erzeugten Hochfrequenzfeldes gesteuert (z.B. geregelt) werden. Beispielsweise kann die Plastizität (die Härte) der erhitzten Schläuche als Maß für die im Schlauchmaterial vorliegende Temperatur angesehen werden, wobei die Plastizität der Schläuche z.B. anhand eines Maßes der zum Klemmen der Schläuche mittels der Klemmvorrichtungen erforderlichen Kraft ermittelt wird (z.B. -wenn die Klemmvorrichtungen per Elektromotor betätigt werden - anhand des vom Motor aufgenommenen Stroms, der erforderlich ist, um die Klemmvorrichtung, insbesondere deren Klemmbacken, in eine Klemmposition zu bringen).

Es ist insbesondere möglich, dass der Motorstrom (bzw. die Motorspannung) vorgegeben wird und mit Hilfe eines Encoders des Motors die Stellung der angetriebenen Klemmbacke überwacht wird, wobei anhand des aufgenommenen Motorstroms festgestellt werden kann, wann die Klemmbacke an dem Schlauch anliegt. Nach Anliegen der Klemmbacke am Schlauch wird die Hochfrequenzspannung eingeschaltet. Je nach Temperatur (d.h. Weichheit) des Schlauches wird sich die Klemmbacke weiter bewegen, wobei z.B. nach einem vorgebbaren Wert für den Weg, den die Klemmbacke nach dem Einschalten der Hochfrequenzspannung zurücklegt, d.h. bei einer bestimmten Weichheit des Schlauches, das Auseinanderreißen des Schlauches initiiert wird (d.h. z.B. die weiter unten beschriebenen Bewegungserzeugungsmittel aktiviert werden).

Des Weiteren beeinflusst die Temperatur des Schlauches die zur Einspeisung der Hochfrequenz erforderliche Anpassung einer Hochfrequenzschaltung der Hochfrequenzspannungserzeugungsmittel, was ebenfalls zur Temperaturregelung genutzt werden kann.

Es wird darauf hingewiesen, dass die Erfindung nicht auf Erhitzen per Hochfrequenzfeld begrenzt ist. Vielmehr können auch andere Erhitzungsmittel eingesetzt werden, insbesondere solche, die ein berührungsloses Erhitzen des Schlauches ermöglichen (z.B. ein Heißlufterzeuger oder ein Laser).

Gemäß einer anderen Weiterbildung der Erfindung ist jeder der Aufnahmevorrichtungen mindestens eine weitere Klemmvorrichtung zum Klemmen eines an der Aufnahmevorrichtung angeordneten Schlauches zugeordnet, die jeweils so an der Aufnahmevorrichtung angeordnet ist, dass sie bei Rotation der Aufnahmevorrichtung mitrotiert. Die zweite Klemmvorrichtung dient insbesondere zum Halten des Schlauches an der jeweiligen Aufnahmevorrichtung und/oder zum Zusammendrücken des Schlauches vor und nach dem Auftrennen. Beispielsweise umfasst diese weitere Klemmvorrichtung ebenfalls zwei Klemmbacken, die mit der Aufnahmevorrichtung verbunden sind. Zumindest eine der beiden Klemmbacken der weiteren Klemmvorrichtung kann aus einem Kunststoff gebildet sein, um eine Beeinflussung eines Hochfrequenzfeldes möglichst zu vermeiden, falls die Erhitzungsmittel auf dem Erhitzen per Hochfrequenzfeld basieren.

Beispielsweise ist eine der beiden Klemmbacken der weiteren Klemmvorrichtung fest mit der Aufnahmevorrichtung verbunden, während die andere relativ zu der Aufnahmevorrichtung bewegbar (z.B. per Elektromotor) mit dieser verbunden ist, so dass durch Bewegen (z.B. Rotieren) der bewegbaren Klemmbacke auf die feste Klemmbacke zu der Schlauch zwischen den beiden Klemmbacken eingeklemmt werden kann.

Insbesondere umfasst die erfindungsgemäße Vorrichtung sowohl eine nicht mit der zugeordneten Aufnahmevorrichtung rotierende (erste) Klemmvorrichtung als auch eine mitrotierende (zweite) Klemmvorrichtung (pro Aufnahmevorrichtung). Beispielsweise sind die ersten und die zweite Klemmvorrichtung so angeordnet, dass der Schlauch jeweils durch eine Relativbewegung der Klemmvorrichtungen zueinander eine Zug- und/oder Scherkraft auf den jeweiligen Schlauch ausgeübt wird, die zum Auftrennen des Schlauches im erhitzten Trennbereich führt. Etwa ist möglich, dass die Erhitzungsmittel den Schlauch im Bereich der beiden Klemmvorrichtungen und/oder im Bereich zwischen den beiden Klemmvorrichtungen erhitzen, so dass der Schlauch jeweils zwischen der ersten und der zweiten Klemmvorrichtung aufgrund deren Relativbewegung zueinander aufgetrennt wird.

Beispielsweise werden durch die zweite Klemmvorrichtung jeweils Innenseiten des Endes des ersten Schlauchabschnitts gegeneinander gedrückt, wodurch aufgrund der hohen Temperatur des Schlauchendes insbesondere eine stoffschlüssige Verbindung der Innenseiten entsteht, so dass das Ende der ersten Schlauchabschnitte jeweils verschlossen wird und das Innere des ersten Schlauchabschnitts somit während des Herstellen des mechanischen Kontaktes zwischen den ersten Schlauchabschnitten steril bleibt. Die erste Klemmvorrichtung verschließt analog den zweiten Schlauchabschnitt.

Das Auftrennen des ersten und des zweiten Schlauches erfolgt, wie oben bereits angedeutet, z.B. dadurch, dass auf die Schläuche eine Zug- und oder Scherkraft ausgeübt wird, d. h. die Auftrennmittel umfassen Mittel zum Ausüben einer Zug- und oder Scherkraft, die zum Auftrennen der Schläuche im erhitzten Trennbereich führt. Beispielsweise umfassen die Auftrennmittel Bewegungserzeugungsmittel zum Erzeugen einer Bewegung der ersten und/oder der zweiten Aufnahmevorrichtung, wobei durch die unter Verwendung der Bewegungserzeugungsmittel erzeugte Bewegung der ersten und/oder der zweiten Aufnahmevorrichtung eine Zug- und/oder Scherkraft in den ersten und/oder zweiten Schlauch eingeleitet wird, die den ersten und/oder den zweiten Schlauch in den ersten und den zweiten Schlauchabschnitt auftrennt.

Die erfindungsgemäße Vorrichtung ist jedoch nicht auf Auftrennmittel, die eine Zug- oder Scherkraft erzeugen, beschränkt. Das Auftrennen der Schläuche kann vielmehr auch auf andere Weise erfolgen, z.B. unter Verwendung eines Lasers oder einer Schneidkante. Auch ist denkbar, dass unterschiedliche Auftrennmittel in Kombination miteinander verwendet werden, z.B. kann einer der Schläuche durch Ausüben einer Zugkraft und der andere Schlauch durch Ausüben einer Scherkraft aufgetrennt werden.

Des Weiteren kann eine Zug- und oder Scherkraft auf die Schläuche auch dadurch erzeugt werden, dass die erste und/oder die zweite Aufnahmevorrichtung um ihre Rotationsachse, die senkrecht zu der Ebene des sich im Bereich der Aufnahmevorrichtung erstreckenden Schlauchabschnitts verläuft, gedreht wird. Mit anderen Worten steuern die Auftrennmittel lediglich die Rotation der Aufnahmevorrichtungen, um eine Auftrennkraft (insbesondere eine Scherkraft) im Trennbereich der Schläuche zu erzeugen.

Gemäß einer Ausgestaltung der Erfindung umfassen die Bewegungserzeugungsmittel ein mit der ersten Aufnahmevorrichtung verbundenen ersten Arm und einen mit der zweiten Aufnahmevorrichtung verbundenen zweiten Arm, wobei die beiden Arme so miteinander verbunden sind, dass sie nach Art einer Schere um eine gemeinsame Rotationsachse relativ zueinander rotierbar sind. Beispielsweise sind ein Endbereich des ersten Armes mit der ersten Aufnahmevorrichtung und ein Endbereich des zweiten Armes mit der zweiten Aufnahmevorrichtung gekoppelt. Ein von dem Endbereich beabstandeter Bereich des ersten Armes überlappt mit einem von dem Ende des zweiten Armes beabstandeten Bereich des zweiten Armes, wobei in diesem Überlappungsbereich z. B. Kopplungsmittel (etwa in Form eines Stiftes) vorgesehen sind, die die beiden Arme relativ zueinander rotierbar miteinander koppeln. Durch Auseinanderspreizen der beiden Arme können die Aufnahmevorrichtungen bewegt werden, und zwar entsprechend der Bewegung der Arme um die Rotationsachse, um die die Arme rotiert werden, d.h. insbesondere um eine Rotationsachse, die außerhalb der Aufnahmevorrichtungen liegt. Die Bewegungserzeugungsmittel ermöglichen somit eine zusätzliche Bewegung der Aufnahmevorrichtungen (zusätzlich zu einer möglichen Rotation um die Rotationsachse der Aufnahmevorrichtungen, die senkrecht zu der Ebene, entlang derer sich die ersten Schlauchabschnitte erstrecken, orientiert ist).

Bei entsprechender Länge der Arme (d.h. des Radius der Kreisbahn, auf der die Aufnahmevorrichtungen bewegt werden), kann eine nahezu lineare Bewegung der Aufnahmevorrichtungen erzeugt werden.

Es ist auch denkbar, dass die Auftrennmittel Translationserzeugungsmittel zum Erzeugen einer Translation (d. h. einer rein geradlinigen oder einer zumindest im Wesentlichen geradlinigen Bewegung) der ersten und/oder der zweiten Aufnahmevorrichtung umfassen, wobei durch eine durch die Translationszeugungsmittel hervorgerufene Translation ebenfalls eine Zug- und/oder Scherkraft auf den ersten und/oder zweiten Schlauch erzeugt und damit das Auftrennen der Schläuche bewirkt wird. Es ist natürlich auch möglich, dass die Auftrennmittel sowohl Rotationserzeugungsmittel als auch Translationserzeugungsmittel aufweisen, d. h. zumindest eine der beiden Aufnahmevorrichtungen kann sowohl eine Rotation als auch eine Translationsbewegung (insbesondere gleichzeitig) ausführen.

Des Weiteren kann die Vorrichtung eine erste Klemmvorrichtung (wie oben bereits beschrieben), die einer der Aufnahmevorrichtungen zugeordnet ist, umfassen, in der der Schlauch festgeklemmt werden kann und die bei einer Rotations- und/oder Translationsbewegung der Aufnahmevorrichtung nicht mitbewegt wird. Des Weiteren umfasst die Vorrichtung (ebenfalls wie oben beschrieben) z.B. eine zweite Klemmvorrichtung, die bei einer Rotation und/oder einer Translation der Aufnahmevorrichtung mitbewegt wird, so dass eine Zug- und/oder Scherkraft durch translatorische und/oder rotatorische Bewegung der Aufnahmevorrichtung relativ zu der (ortsfesten) ersten Klemmvorrichtung erzeugt werden kann. Nach Auftrennen des Schlauches klemmt dann die erste Klemmvorrichtung das entstandene freie Ende des zweiten Schlauchabschnitts und die zweiten Klemmvorrichtung das freie Ende des ersten Schlauchabschnitts.

Gemäß einer weiteren Ausführungsform der Erfindung sind die erste und/oder die zweite Aufnahmevorrichtung jeweils plattenartig ausgestaltet, insbesondere in Form einer Scheibe (d. h. in Form einer in Draufsicht zumindest näherungsweise kreisförmig ausgeformten Platte), ausgebildet. Beispielsweise ist die Scheibe um eine Rotationsachse rotierbar, die durch ihren Mittelpunkt hindurch verläuft.

Nach einer anderen Ausgestaltung der Erfindung umfasst die erste und/oder die zweite Aufnahmevorrichtung jeweils einen Aufnahmekörper, der in einer parallel zur Rotationsachse (die der jeweiligen Aufnahmevorrichtung zugeordnet ist) orientierten Seite eine Aussparung (z. B. in Form einer Nut) zum Einlegen des ersten oder des zweiten Schlauches aufweist. Beispielsweise ist die Aufnahmevorrichtung in Form einer Platte ausgestaltet, wobei der Aufnahmekörper als prismenartiger Körper an einer der Grundflächen der Platte angeordnet ist. Die erwähnte Aussparung befindet sich dann z. B. in einer senkrecht zur Grundfläche der Platte verlaufenden Seite des prismenartigen Körpers.

Die oben beschrieben Vorrichtung dient insbesondere dazu, ein Verfahren zum sterilen Verbinden von Schläuchen auszuführen, das die folgenden Schritte aufweist:
- Bereitstellen eines ersten und eines zweiten Schlauchs;
- Erhitzen des ersten und des zweiten Schlauchs in einem Trennbereich;
- Auftrennen des ersten und des zweiten Schlauchs in dem erhitzten Trennbereich in einen ersten und einen zweiten Schlauchabschnitt; und
- Herstellen eines mechanischen Kontaktes eines Endes der ersten Schlauchabschnitts des ersten Schlauches mit einem Ende des ersten Schlauchabschnitts des zweiten Schlauches,
- wobei das Herstellen des mechanischen Kontaktes unter Rotieren des ersten und/oder zweiten Schlauchabschnitts um eine Rotationsachse erfolgt, die zumindest näherungsweise senkrecht zu einer Ebene verläuft, entlang derer sich jeweils der erste Schlauchabschnitt zumindest teilweise erstreckt.

Die Erfindung wird im Folgenden anhand von Ausführungsbeispielen unter Bezugnahme auf die Figuren näher erläutert. Es zeigen:
- Figuren 1A - 1D: schematisch Verfahrensschritte eines Verfahrens gemäß einem Ausführungsbeispiel der Erfindung;
- Figuren 2A - 2H: das Funktionsprinzip einer Vorrichtung gemäß einem ersten Ausführungsbeispiel der Erfindung;
- Figuren 3A - 3F: das Funktionsprinzip von Bewegungserzeugungsmitteln;
- Figuren 4A - 4G: das Funktionsprinzip einer Abwandlung der Vorrichtung aus den Figuren 2A- 2H;
- Figuren 5A - 5E: Schritte beim Zentrieren von an einer Aufnahmevorrichtung der erfindungsgemäßen Vorrichtung angeordneten Schläuchen unterschiedlichen Durchmessers;
- Figuren 6A - 6F: das Positionieren von ersten Schlauchabschnitten;
- Figuren7A - 7F: eine erste Ausgestaltung von Klemmvorrichtungen der erfindungsgemäßen Vorrichtung; und
- Figuren 8A - 8F: eine zweite Ausgestaltung von Klemmvorrichtungen der erfindungsgemäßen Vorrichtung.

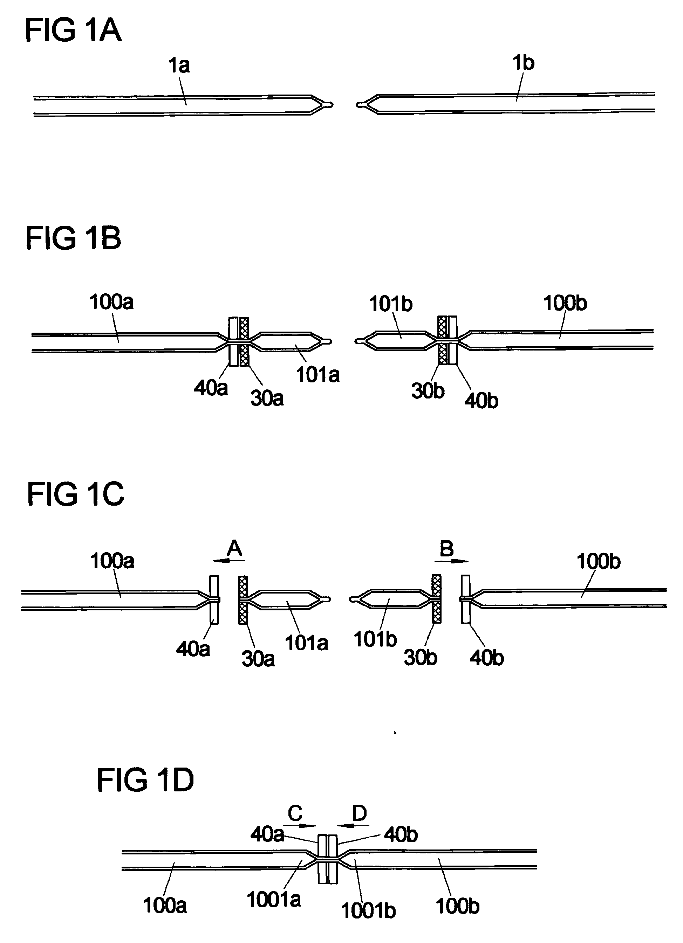

Figuren 1A bis 1D veranschaulichen ein Verfahren zum sterilen Verbinden von Schläuchen gemäß einem Ausführungsbeispiel der Erfindung. Danach werden ein erster und ein zweiter Schlauch 1 a, 1 b, die jeweils aus einem thermoplastischen Material gebildet sind, bereitgestellt, die miteinander verbunden werden sollen. Die Schläuche 1 a, 1 b weisen jeweils ein geschlossenes Ende auf und sind mit ihrem jeweils anderen Ende (nicht dargestellt) z.B. mit einem Flüssigkeitsreservoir verbunden (Figur 1A).

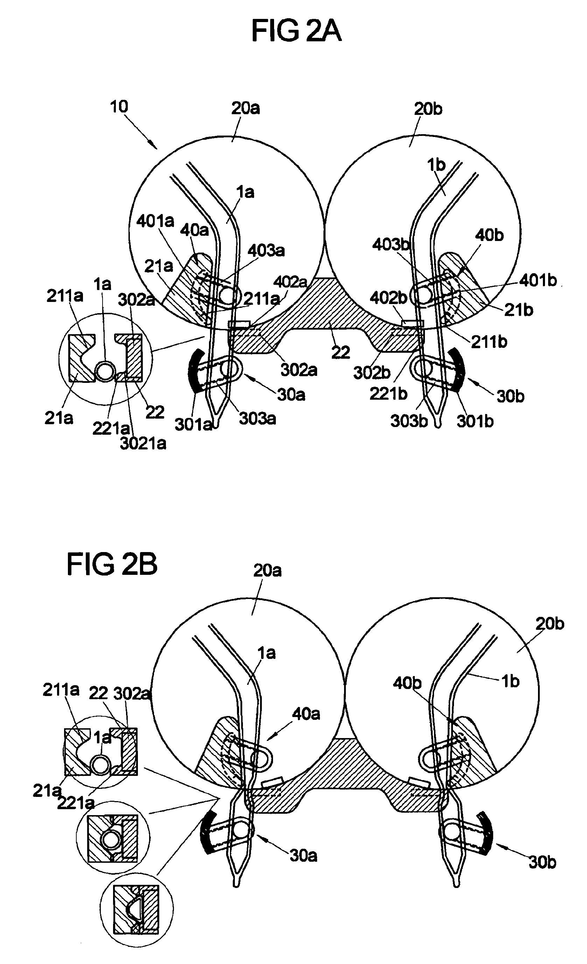

Vor dem Verbinden der Schläuche 1a, 1 b erfolgt jeweils ein Auftrennen der Schläuche in einen ersten Schlauchabschnitt 100a, 100b und einen zweiten Schlauchabschnitt 101 a, 101 b (der das freie Ende des jeweiligen Schlauches aufweist). Zur Vorbereitung der Auftrennung der Schläuche werden sie jeweils mittels einer ersten Klemmvorrichtung 30a, 30b und einer zweiten Klemmvorrichtung 40a, 40b geklemmt. Unter Verwendung von Erhitzungsmitteln in Form von Hochfrequenzspannungserzeugungsmitteln (nicht dargestellt) wird eine hochfrequente elektrische Spannung an die ersten Klemmvorrichtungen 30a, 30b geleitet, so dass im Bereich der ersten Klemmvorrichtungen 30a, 30b ein hochfrequentes elektrisches Feld entsteht, das die Schläuche 1a, 1 b jeweils in einem vorgesehenen Trennbereich, d.h. insbesondere im Bereich der ersten Klemmvorrichtungen 30a, 30b sowie in einem Bereich zwischen den ersten und den zweiten Klemmvorrichtungen 30a, 30b bzw. 40a, 40b, erhitzt (z.B. auf mindestens 200°C). Insbesondere reißen die Schläuche im Bereich zwischen den ersten und den zweiten Klemmvorrichtungen 30a, 30b bzw. 40a, 40b auseinander, was weiter unten in Bezug auf die Figuren 2A bis 2H noch erläutert wird. Somit ermöglicht das erfindungsgemäße Verfahren und entsprechend die erfindungsgemäße Vorrichtung ein berührungsloses Auftrennen der Schläuche.

Nachdem der Trennbereich der Schläuche 1 a, 1 b jeweils auf eine Temperatur oberhalb des Schmelzpunktes des Schlauchmaterials erhitzt wurde, d.h. sich das Schlauchmaterial im Trennbereich im thermoplastischen Zustand befindet, wird jeweils eine Zugkraft entlang ihrer Längsachse auf die Schläuche 1a, 1 b ausgeübt, so dass sie im erweichten Trennbereich auseinandergerissen werden (Figur 1 C). Die Zugkraft wird im Beispiel der Figuren 1A bis 1D dadurch erzeugt, dass die Klemmvorrichtungen 30a, 30b jeweils ortsfest gelagert sind und auf die Klemmvorrichtungen 40a, 40b eine Kraft weg von der Klemmvorrichtung 30a bzw. 30b ausgeübt wird (in Figur 1C angedeutet durch die Pfeile A, B).

Beispielsweise werden die Erhitzungsmittel nach dem Auftrennen der Schläuche 1a, 1 b (oder kurz vorher) abgeschaltet, d. h. das Einleiten der Hochfrequenzspannung in die Klemmvorrichtungen 30a, 30b wird beendet. Es ist jedoch auch denkbar, dass die Erhitzungsmittel nach dem Auftrennen der Schläuche eingeschaltet bleiben, z.B. um die Restschlauchabschnitte 101 a, 101 b, in denen sich Flüssigkeit befinden kann, sicher zu verschließen. Nach dem Auftrennen der Schläuche 1a, 1 b wird ein mechanischer Kontakt zwischen dem durch das Auftrennen des ersten Schlauches 1 a gebildeten Ende 1001 a des ersten Schlauchabschnitts 100a und dem durch das Auftrennen des zweiten Schlauches 1 b gebildeten Ende 1001 b des ersten Schlauchabschnitts 100b des zweiten Schlauches 1 b hergestellt. Hierzu werden insbesondere die nun separaten ersten Schlauchabschnitte 100a, 100b zunächst von den zweiten Schlauchabschnitten 101 a, 101 b wegbewegt und entlang einer gemeinsamen Achse ausgerichtet. Abschließend werden die ersten Schlauchabschnitte 100a, 100b aufeinander zu bewegt (Pfeile C, D), bis ihre beiden Enden 1001 a, 1001 b in mechanischen Kontakt miteinander kommen (Figur 1D).

Es wird darauf hingewiesen, dass die Erhitzungsmittel auch so lange eingeschaltet bleiben können, bis die Schlauchenden 1001 a, 1001 b miteinander verbunden sind. Allerdings sind die Enden 1001 a, 1001 b der ersten Schlauchabschnitte 100a, 100b nach dem Auftrennen der Schläuche beabstandet von den Erhitzungsmitteln (insbesondere von den HF-Klemmvorrichtungen 30a, 30b) positioniert, so dass durch die Erhitzungsmittel kein Erhitzen der Enden 1001 a, 1001 b erfolgt.

Das Herstellen des mechanischen Kontaktes zwischen den Enden 1001 a, 1001 b nach dem Auftrennen der Schläuche 1 a, 1 b erfolgt so schnell, dass die beiden Enden 1001 a, 1001 b jeweils noch eine Temperatur oberhalb des Schmelzpunktes des Schlauchmaterials aufweisen, wenn sie miteinander in Berührung kommen, so dass sie eine stoffschlüssige Verbindung miteinander eingehen (d. h. miteinander verschweißen), ohne dass ein erneutes Erhitzen der Enden 1001 a, 1001 b erforderlich ist.

Die Figuren 2A bis 2H zeigen jeweils verschiedene Schritte bei der Herstellung einer sterilen Verbindung zwischen zwei Schläuchen unter Verwendung einer Vorrichtung gemäß einem ersten Ausführungsbeispiel der Erfindung. Gemäß Figur 2A weist die Vorrichtung 10 eine erste Aufnahmevorrichtung in Form einer ersten Scheibe 20a sowie eine zweite Aufnahmevorrichtung in Form einer zweiten Scheibe 20b auf. Die Scheiben 20a, 20b sind jeweils um eine Rotationsachse rotierbar gelagert, die sich senkrecht zu ihren jeweiligen Grundflächen und durch ihren Mittelpunkt hindurch erstreckt.

An der ersten Scheibe 20a ist ein erster Schlauch 1 a aufgenommen, an der zweiten Scheibe 20b ein zweiter Schlauch 1 b. Die Schläuche 1a, 1 b sind jeweils entlang eines an der jeweiligen Scheibe 20a, 20b angeordneten Aufnahmekörpers 21 a, 21 b geführt, wobei die Aufnahmekörper 21 a, 21 b zur Führung des Schlauches jeweils eine Nut 211a, 211b aufweisen (vgl. den links unten neben der Hauptzeichnung in Figur 2A im Querschnitt dargestellten Ausschnitt der Vorrichtung 10), die sich zumindest über einen Teil einer dem Schlauch 1 a bzw. 1 b zugewandten Seite des prismenartigen Aufnahmekörpers 21 a, 21 b erstreckt.

Die Vorrichtung 10 umfasst des Weiteren einen beiden Scheiben 20a, 20b gemeinsam zugeordneten (jedoch separat zu ihnen angeordneten) Führungskörper 22, der eine erste, dem ersten Schlauch 1 a zugewandte Nut 221 a und eine zweite, dem zweiten Schlauch 1 b zugewandte zweite Nut 221 b aufweist, die zur Führung des jeweiligen Schlauches dienen; vgl. die bereits oben erwähnte Querschnittsdarstellung eines Ausschnittes der Vorrichtung 10 in Figur 2A.

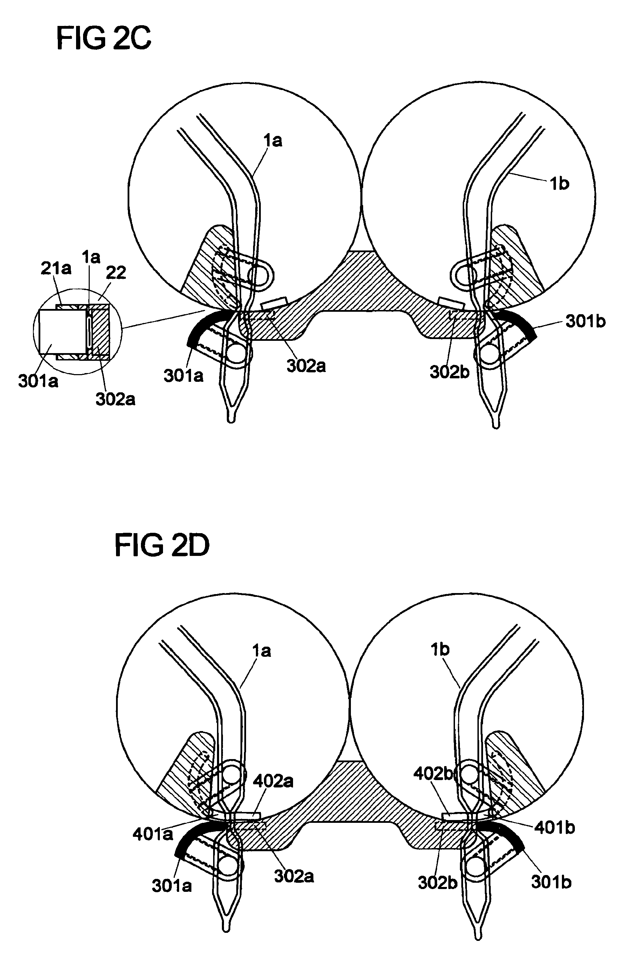

Darüber hinaus weist die Vorrichtung 10 zwei erste Klemmvorrichtungen 30a, 30b auf, die jeweils der ersten Scheibe 20a bzw. der zweiten Scheibe 20b zugeordnet sind. Die ersten Klemmvorrichtungen 30a, 30b besitzen jeweils eine erste Klemmbacke 301 a, 301 b, die um eine Rotationsachse rotierbar gelagert sind, die senkrecht zu der Grundfläche der Scheibe 20a, 20b (d. h. auch senkrecht zu einer Ebene oder Längsachse, entlang derer sich der Schlauch 1 a, 1 b zumindest im Bereich der Aufnahmekörper 21 a, 21 b erstreckt) verläuft. Zwischen den Klemmbacken 301 a, 302a bzw. 301 b, 302b werden die Schläuche 1 a, 1 b durch Rotation der ersten Klemmbacken 301 a, 301 b eingeklemmt (vgl. die weiter unten genauer erläuterte Figur 2C).

Die Vorrichtung 10 umfasst zudem zwei zweite Klemmvorrichtungen 40a, 40b, die ebenfalls jeweils einer der Scheiben 20a, 20b zugeordnet sind. Die zweiten Klemmvorrichtungen 40a, 40b weisen jeweils eine an der Scheibe 20a, 20b angeordnete, aber relativ zu der jeweiligen Scheiben 20a, 20b rotierbare erste Klemmbacke 401 a, 401 b auf sowie eine fest (nicht rotierbar) an den Scheiben 20a, 20b angeordnete zweite Klemmbacke 402a, 402b. Durch Rotation der jeweils ersten Klemmbacken 401 a, 401 b in Richtung auf die zweiten Klemmbacken 402a, 402b kann der Schlauch oberhalb der ersten Klemmvorrichtung (d.h. auf einer dem freien Ende der Schläuche 1a, 1 b abgewandten Seite der ersten Klemmvorrichtung 30a, 30b) geklemmt werden. Beispielsweise sind die Klemmbacken 401 a, 401 b innerhalb einer entsprechend ausgestalteten Aussparung in dem jeweiligen Aufnahmekörper 21 a, 21 b geführt.

Nach Einlegen der Schläuche 1 a, 1 b in die jeweilige Nut 211 a, 221 a bzw. 211 b, 221 b der Aufnahmekörper 21 a, 21 b und dem Führungskörper 22 werden die Scheiben 20a, 20b etwas auf den Führungskörper 22 zu rotiert, d. h. die erste Scheibe 20a rotiert gegen, die zweite Scheibe 20b im Uhrzeigersinn, so dass die Aufnahmekörper 40a, 40b auf den Führungskörper 22 zu bewegt werden, wodurch der Schlauch 1a, 1 b etwas zwischen den Aufnahmekörpern 40a, 40b und der dem jeweiligen Schlauch 1 a, 1 b zugeordneten Nut 221 a, 221 b des Führungskörpers 22 geklemmt wird.

Durch die zumindest näherungsweise V- oder U-förmige Ausgestaltung zumindest einer der Nuten 211a, 211b bzw. 221 a, 221 b wird der Schlauch zentriert, d. h. aus seiner Position nach dem Einlegen etwas von den Scheiben 20a, 20b (vertikal) weg bewegt, so dass der Mittelpunkt des zwischen den Aufnahmekörpern 40a, 40b und dem Führungskörper 22 geklemmten Abschnitts der Schläuche 1 a, 1 b unabhängig von dem Durchmesser des Schlauches einen definierten Abstand zu der Oberseite der Scheiben 1a, 1 b aufweist, d.h. in Bezug auf die Nuten 211 a, 211 b bzw. 221 a, 221 b zentriert wird. Dieses Zentrieren der Schläuche ermöglicht insbesondere ein Verbinden von Schlauchabschnitten unterschiedlichen Durchmessers, worauf weiter unten noch näher eingegangen werden wird. Der Zentriervorgang ist insbesondere in den Querschnittsdarstellungen links neben der Hauptzeichnung der Figur 2B dargestellt, wobei zu beachten ist, dass der Aufnahmekörper 211 a und der Führungskörper 22 entlang des Schlauches 1a hintereinander angeordnet sind.

Nach dem anfänglichen Rotieren der Scheiben 20a, 20b gemäß Figur 2B erfolgt ein Rotieren jeweils der ersten Klemmbacke 301 a, 301 b der ersten Klemmvorrichtungen 30a, 30b auf den jeweiligen Schlauch 1 a, 1 b zu, bis der Schlauch 1 a, 1 b zwischen den beiden Klemmbacken 301 a, 302a, bzw. 301 b, 302b eingeklemmt ist; vgl. Figur 2C. Anschließend werden auch die ersten Klemmbacken 401 a, 401 b der zweiten Klemmvorrichtung 40a, 40b aktiviert, so dass sie ebenfalls in Richtung Schlauch schwenken und den Schlauch 1a, 1 b gegen die jeweils zweiten Klemmbacken 402a, 402b drücken und somit klemmen; vgl. Figur 2D. Die Schwenkbewegung der ersten Klemmbacken 301 a, 301 b und 401 a, 401 b wird insbesondere über Elektromotoren erzeugt, wobei insbesondere jeder Klemmbacke ein Elektromotor zugeordnet ist. Beispielsweise sind die Elektromotoren auf einer Rückseite (d.h. einer den Schläuchen 1a, 1 b abgewandten Seite) der Klemmbacken angeordnet. Die Klemmbacken sind insbesondere über Arme 303a, 303b, 403a, 403b schwenkbar mit einer Welle des jeweiligen Motors verbunden.

Gleichzeitig oder vor der Schwenkbewegung der ersten Klemmbacken 401 a, 401 b erfolgt eine Rotation der Scheiben 20a, 20b zumindest näherungsweise zurück in ihre in Figur 2A gezeigte Ausgangsstellung. Die ersten Klemmvorrichtungen 30a, 30b werden vor den zweiten Klemmvorrichtungen 40a, 40b aktiviert, insbesondere, um einem Druckaufbau in den Restschlauchabschnitten 101 a, 101 b, der zu einem Aufreißen oder Platzen der Restschlauchabschnitte führen könnte, entgegenzuwirken. Es ist natürlich dennoch auch denkbar, dass die zweiten Klemmvorrichtungen 40a, 40b vor den ersten Klemmvorrichtungen 30a, 30b aktiviert werden.

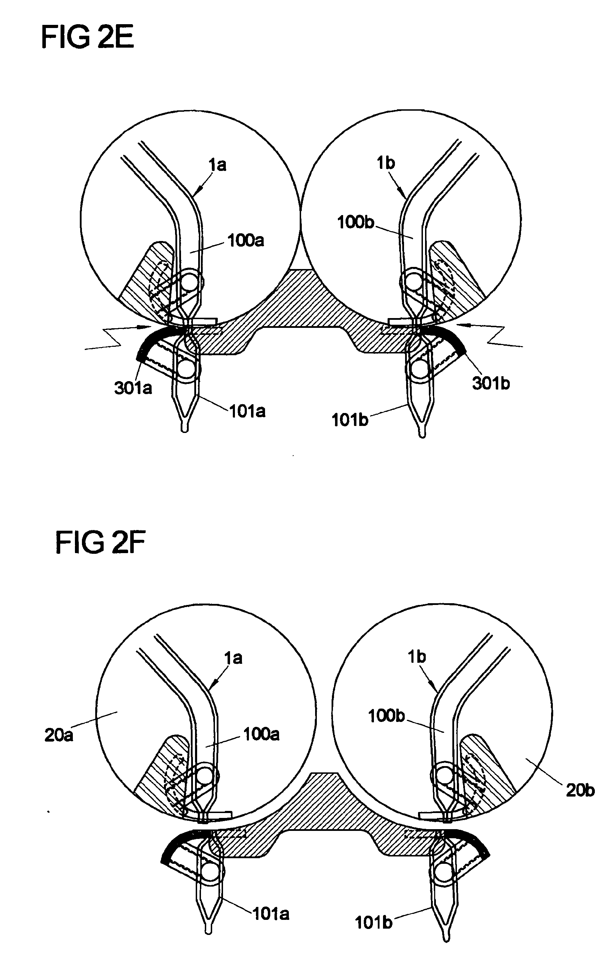

Die Vorrichtung 10 umfasst des Weiteren Erhitzungsmittel zum Erhitzen eines Trennbereiches der Schläuche 1a, 1 b, in dem jeweils ein erster Schlauchabschnitt in Form des Hauptschlauchabschnitts 100a, 100b und ein zweiter Schlauchabschnitt in Form eines Endschlauchabschnitts 101 a, 101 b aneinander grenzen. Die Erhitzungsmittel sind in Form von Hochfrequenzspannungserzeugungsmitteln (insbesondere umfassend eine Hochfrequenzspannungsquelle, die in den Figuren 2A - 2H nicht dargestellt ist) ausgebildet, die mit den zweiten, metallischen Klemmbacken 302a, 302b ("Heißelektroden") der ersten Klemmvorrichtungen 30a, 30b elektrisch verbunden sind und die nach Aktivieren eine Hochfrequenzspannung in die Klemmbacken 302a, 302b einleiten (in Figur 2E angedeutet durch blitzartige Pfeile).

Die ersten, bewegbaren Klemmbacken 301 a, 301 b befinden sich insbesondere auf Massepotential. Die HF-Klemmbacken 302a, 302b sind in einer an die Nuten 221 a, 221 b angrenzenden Aussparung in dem Führungskörper 22 angeordnet, der z.B. zumindest teilsweise aus einem elektrisch isolierenden Material gebildet sein kann. Die HF-Klemmbacken 302a, 302b sind zudem so positioniert, dass sie jeweils mit einer im Wesentlichen planen Klemmseite 3021 a, 3021 b, mit der sie in Klemmstellung der Klemmvorrichtungen 30a, 30b an dem Schlauch anliegen und die Hochfrequenzspannung in den Schlauch einleiten, an die Nut 221 a, 221 b angrenzen, d.h. die Klemmseiten 3021 a, 3021 b bilden einen Boden der jeweiligen Nut.

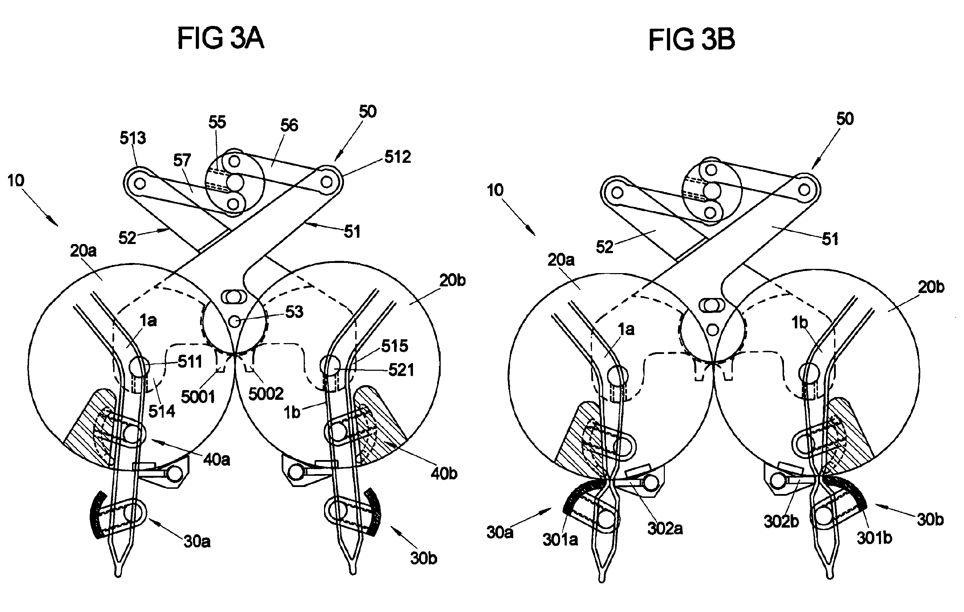

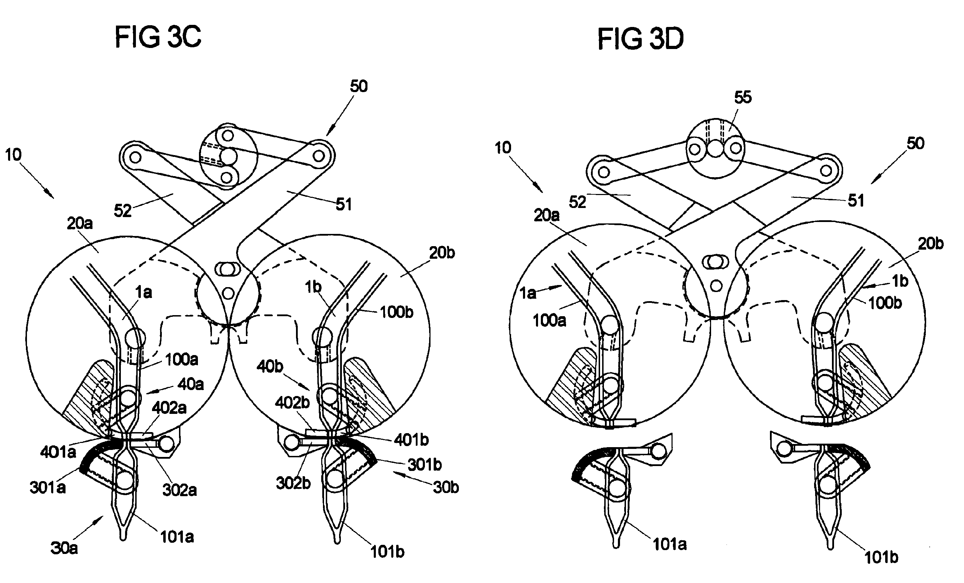

Die Hochfrequenzspannungserzeugungsmittel erzeugen über die Klemmbacken 301 a, 302a, 301 b, 302b ein elektrisches Hochfrequenzfeld im Trennbereich der Schläuche 1a, 1 b (d. h. in einem Bereich in der Nähe der Klemmbacken 301 a, 301), das die Schläuche jeweils in diesem Trennbereich erhitzt. Nach dem Erhitzen des Trennbereiches bzw. während des Erhitzens wird eine Bewegung der Scheiben 20a, 20b von den ersten Klemmvorrichtungen 30a, 30b weg initiiert (vgl. Figur 2F). Zum Erzeugen dieser Bewegung verfügt die Vorrichtung 10 über Bewegungserzeugungsmittel, die beispielhaft anhand der Figuren 3A - 3F erläutert werden.

Durch die Bewegung der Scheiben 20a, 20b von den ersten Klemmvorrichtungen 30a, 30b weg wird eine Zug- und Scherkraft auf die Schläuche 1 a, 1 b ausgeübt, so dass sie im Trennbereich auseinander gezogen werden und schließlich auseinanderreißen, d.h. die Hauptschlauchabschnitte 100a, 100b werden von den Endschlauchabschnitten 101 a, 101 b getrennt. Es wird darauf hingewiesen, dass die in Figur 2F dargestellte Bewegung der Scheiben 20a, 20b nicht zwingend erforderlich ist, um das Auftrennen der Schläuche zu bewirken. Vielmehr ist auch denkbar, dass eine Trennkraft dadurch erzeugt wird, dass die Scheiben 20a, 20b jeweils lediglich in Rotation um ihre durch ihren Mittelpunkt verlaufenden Rotationsachse versetzt werden, ohne dass die Scheiben 20a, 20b von dem Führungskörper 22 weg bewegt werden.

Zwischen den Schlauchabschnitten 100a, 101 a bzw. 100b, 101 b entsteht insbesondere eine glatte Abrisskante oberhalb der ersten und der zweiten Klemmbacke 301 a, 302a, 301 b, 302b (d.h. der "heißen" HF-Elektroden), was insbesondere darauf zurückzuführen ist, dass über die aus Metall gebildeten ersten Klemmbacken 301 a, 301 b ein besserer Wärmetransport vom Schlauch 1 a, 1 b erfolgt als im Bereich oberhalb der Klemmbacken 301 a, 301 b (d. h. im Bereich zwischen den Klemmbacken 301 a, 301 b und den ersten Klemmbacken 401 a, 401 b der zweiten Klemmvorrichtung 40a, 40b), wodurch im Schlauch auf Höhe der Oberseite (d.h. einer der zweiten Klemmvorrichtung 40a, 40b zugewandten Seite) der ersten Klemmbacken 401 a, 401 b ein Temperaturgradient auftritt, wobei unter Einwirkung der Zug- oder Scherkraft das Auseinanderreißen der Schläuche bevorzugt im Bereich dieses Temperaturgradienten erfolgt. Insbesondere besteht der Temperaturgradient an der Oberfläche der Schläuche, wobei es möglich ist, dass die Oberfläche der Schläuche zwischen den Klemmvorrichtungen 30a, 30b und 40a, 40b eine höhere Temperatur aufweist als die Oberfläche der Schläuche im Bereich der ersten Klemmvorrichtungen 30a, 30b (d.h. zwischen den Klemmbacken 301 a, 302a bzw. 301 b, 302b).

Insbesondere weisen die als Hochfrequenzelektroden dienenden Klemmbacken eine flache Seite auf, mit der sie in Klemmstellung an dem Schlauch anliegen, um einerseits eine möglichst gleichmäßiges Feld im Schlauch zu erzeugen und andererseits eine möglichst gute Wärmeableitung vom Schlauch weg zu realisieren.

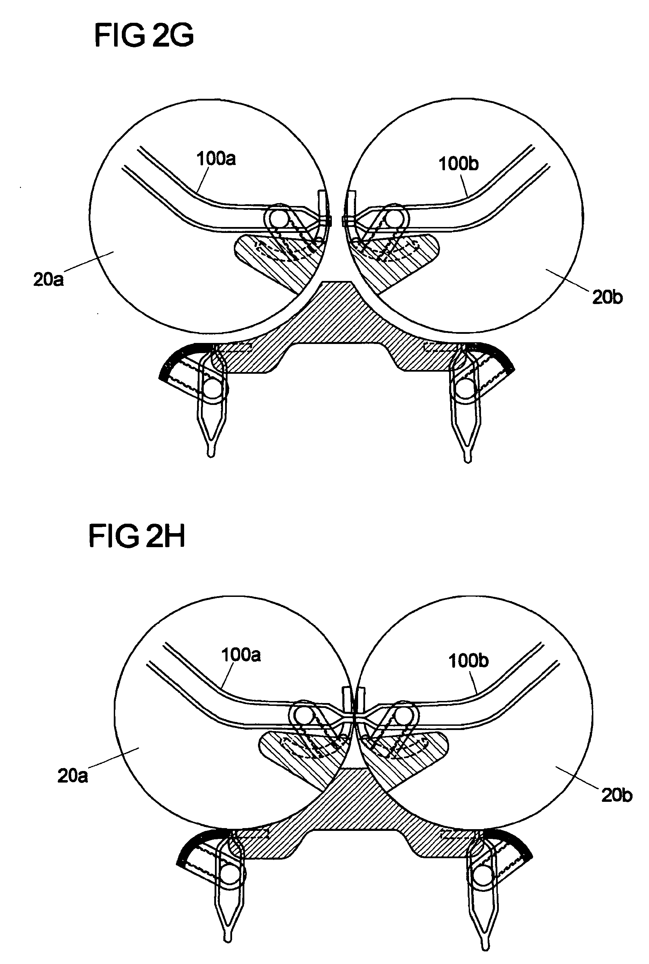

Nach dem Auftrennen der Schläuche 1 a, 1 b in die Hauptschlauchabschnitte 100a, 100b und die Endschlauchabschnitte 101 a, 101 b erfolgt ein Rotieren der Scheiben 20a, 20b derart, dass die durch das Auftrennen der Schläuche 1 a, 1 b entstandenen und von den zweiten Klemmvorrichtungen 40a, 40b gehaltenen Enden der Hauptschlauchabschnitte 100a, 100b aufeinander zu bewegt werden; vergleiche Figur 2G. Hierzu wird die erste Scheibe 20a um die senkrecht zu ihren Grundflächen und damit auch senkrecht zu einer Ebene, entlang derer sich ein Teil (der im Bereich des Aufnahmekörpers 40a verläuft) des Hauptschlauchabschnitts 100a erstreckt, orientierte Rotationsachse gegen den Uhrzeigersinn gedreht. Eine analoge Rotationsbewegung führt die zweite Scheibe 20b aus, allerdings im Uhrzeigersinn.

Die Scheiben 20a, 20b werden jeweils um näherungsweise 90 gedreht, so dass sich die beiden Enden der Hauptschlauchabschnitte 100a, 100b nach der Rotation der Scheiben 20a, 20b entlang einer gemeinsamen Längsachse gegenüberliegen. Anschließend erfolgt eine Bewegung der beiden Scheiben 20a, 20b aufeinander zu, so dass die sich entlang der gemeinsamen Längsachse einander gegenüberliegenden Stirnseiten der Enden der Hauptschlauchabschnitte 100a, 100b berühren (Figur 2H). Insbesondere erfolgt die Rotation der Scheiben 20a, 20b um ihre jeweilige Rotationsachse und die Bewegung der Scheiben aufeinander zu so schnell, dass die Enden der Hauptschlauchabschnitte 100a, 100b infolge des Erhitzens gemäß Figur 2E auch nach Abschaltung der Erhitzungsmittel noch eine derartige Temperatur aufweisen, dass sie, wenn sie gemäß Figur 2H in mechanischem Kontakt miteinander kommen, eine stoffschlüssige Verbindung miteinander eingehen, d. h. miteinander verschweißen, ohne dass hierfür ein erneutes Erhitzen erforderlich ist.

Nach einer Abkühlphase (z.B. mindestens 4 s) können auch die zweiten Klemmbacken 401 a, 401 b der zweiten Klemmvorrichtung 40a, 40b gelöst und der durch Verbinden der Hauptschlauchabschnitte 100a, 100b hergestellte Schlauch aus der Vorrichtung 10 entnommen werden. Die Erfindung ist natürlich nicht auf eine bestimmte Abkühlzeit beschränkt; es können z.B. in Abhängigkeit von der Schlauchbeschaffenheit auch kürzere oder längere Abkühlzeiten erforderlich sein. Insbesondere muss der Schlauch nach der Abkühlzeit eine ausreichende Festigkeit aufweisen, damit er sicher aus der Vorrichtung entnommen und verwendet werden kann.

Durch die hohe Temperatur der Enden der Hauptschlauchabschnitte 100a, 100b bildet sich insbesondere eine Materialhaut, die den Querschnitt der Hauptschlauchabschnitte 100a, 100b verschließt (analog entsteht auch ein Verschluss der Endschlauchabschnitte 101 a, 101 b). Dies wird durch das Klemmen des Schlauches mittels der ersten und der zweiten Klemmvorrichtungen unterstützt, wodurch insbesondere eine Verbindung zwischen Innenseiten der geklemmten (und erhitzte) Schlauchabschnitte entsteht. Dadurch sind die Hauptschlauchabschnitte während des Herstellens des mechanischen Kontaktes insbesondere steril verschlossen. Nach dem Verbinden der Hauptschlauchabschnitte 100a, 100b kann dieser Verschluss fortbestehen, der jedoch durch leichten (insbesondere manuellen) Druck von außen auf die Verbindungsstelle geöffnet werden kann. Durch das Fortbestehen des Verschlusses auch nach dem Verbinden der ersten Schlauchabschnitte besteht die Möglichkeit, zunächst zu überprüfen, ob tatsächlich die richtigen Schläuche (d.h. die richtigen mit diesen Schläuchen verbundenen Behälter) verbunden wurden. Dies kann beispielsweise unter Einscannen von Etiketten der verbundenen Behälter geprüft werden. Erst wenn diese Prüfung bestätigt, dass die richtigen Behälter verbunden wurden, wird der Verschluss zwischen den Schlauchabschnitten durch Druck auf die Verbindungsstelle geöffnet.

Die Figuren 3A bis 3F zeigen eine Ausführungsform einer Vorrichtung 10, die Bewegungserzeugungsmittel zum Erzeugen einer Bewegung der Scheiben 20a, 20b aufweist, um diese, wie in der Figur 2F gezeigt, von den ersten Klemmvorrichtungen 30a, 30b zu entfernen und sie nach Ausrichtung der Enden der Hauptschlauchabschnitte aufeinander zu zu bewegen, wie in Figur 2H illustriert.

Danach umfasst die Vorrichtung Bewegungserzeugungsmittel 50, die eine Rotation der Scheiben 20a, 20b um eine außerhalb der Scheiben verlaufende Rotationsachse generieren. Die Bewegungserzeugungsmittel 50 weisen einen ersten Arm 51 auf, der über Verbindungsmittel 511 (z. B. in Form einer Schraube) zumindest näherungsweise mit einem Mittelpunkt der ersten Scheibe 20a verbunden ist, sowie einen zweiten Arm 52, der über analoge Verbindungsmittel 521 mit dem Mittelpunkt der zweiten Scheibe 20b verbunden ist.

Die beiden Arme 51, 52 sind nach Art einer Schere so miteinander gekoppelt, dass sie um eine gemeinsame Rotationsachse 53 relativ zueinander rotierbar sind. Hierfür erstreckt sich durch miteinander fluchtende Öffnungen der Arme 51, 52 ein Stift. Somit kann durch Auseinanderspreizen von den Scheiben 20a, 20b abgewandten Enden 512, 513 der Arme 51, 52 eine Bewegung der mit den Scheiben 20a, 20b verbundenen Enden 514, 515 der Arme 51, 52 und damit eine Bewegung der Scheiben 20a, 20b erzeugt werden.

Durch die rotierbare Lagerung um den Drehpunkt 53 wird eine Rotationsbewegung der Scheiben 20a, 20b (voneinander weg oder aufeinander zu) auf einer Kreisbahn um die Rotationsachse 53 erzeugt. Das Auseinanderspreizen der den Scheiben 20a, 20b abgewandten Enden 512, 513 der Arme 51, 52 erfolgt über eine rotierbare Ringscheibe 55, die über Verbindungselemente (z. B. näherungsweise stangenförmig ausgebildet) 56, 57 mit den Enden 512, 513 der Arme 51, 52 verbunden ist. Dabei ist ein Ende des ersten Verbindungselementes 57 mit dem Ende 513 des einen Armes 52 drehbar verbunden, während das andere Ende des Verbindungselementes 57 drehbar und exzentrisch an der Ringscheibe 55 befestigt ist. Analog ist das zweite Verbindungselement 56 mit dem Arm 51 und der Ringscheibe 55 gekoppelt, wobei sich die Verbindungsstellen (die z. B. durch eine stiftartige Verbindung der Ringscheibe 55 und der Verbindungselemente 56, 57 realisiert sind) der Verbindungselemente mit der Ringscheibe einander entlang eines Radius der Ringscheibe 55 gegenüberliegen.

Die Ringscheibe wird z.B. über einen Elektromotor angetrieben, dessen Welle insbesondere in einer zentralen Öffnung der Ringscheibe mit dieser verbunden ist. Der Elektromotor befindet sich z.B. auf einer Seite der Ringscheibe, die den Scheiben 20a, 20b abgewandt ist. Somit werden insbesondere sämtliche Elemente, die beim Herstellen der Schlauchverbindung bewegt werden (die Mittel 50, die Scheiben 20a, 20b sowie die bewegbaren Klemmbacken der Klemmvorrichtungen 30a, 30b, 40a, 40b), per Elektromotor angetrieben. Beispielsweise sind sämtliche Elektromotoren mit einem Encoder ausge-stattet, so dass jeweils die Position der bewegten Elemente bestimmt werden kann. Beispielsweise sind die Scheiben 20a, 20b und die Klemmvorrichtungen 30a, 30b, 40a, 40b (zumindest teilweise) auf einer Seite eines Trägers (z.B. in Form einer Metall- oder Kunststoffplatte) angeordnet, während z.B. die Elektromotoren auf einer abgewandten Seite des Trägers angeordnet sind, wobei jeweils eine Welle der Motoren den Träger durchgreift.

Die Figur 3A entspricht der Ausgangsstellung der Vorrichtung 10 (entsprechend der Figur 2A). Die Figur 3B zeigt das Klemmen des Schlauches mittels der ersten Klemmvorrichtungen 30a, 30b analog zu Figur 2C. Die Bewegungszeugungsmittel 50 befinden sich hierbei noch in ihrer Ausgangsposition. Erst nach dem Aktivieren auch der zweiten Klemmvorrichtungen 40a, 40b (analog Figur 2E) und einem Erhitzen der Schläuche 1 a, 1 b wird die Ringscheibe 55 gedreht, um ein Auseinanderspreizen der oberen Enden 512, 513 der Arme 51, 52 und damit ein Auseinanderspreizen der mit den Scheiben 20a, 20b verbundenen Enden 514, 515 der Arme 51, 52 zu erzeugen (Figur 3D).

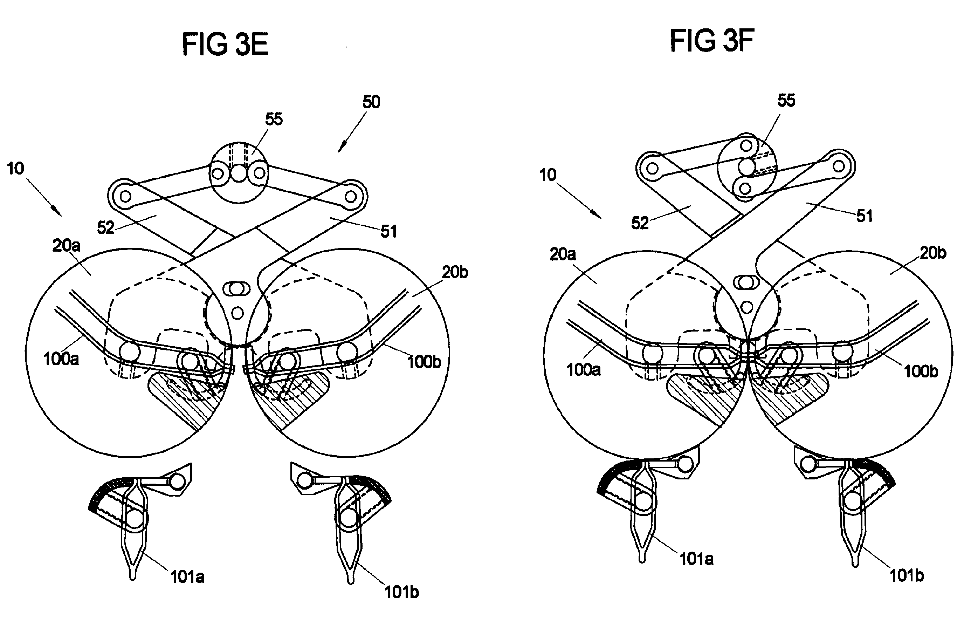

Durch dieses Auseinanderspreizen der unteren Enden 514, 515 bewegen sich die Scheiben auf einer Kreisbahn um den Drehpunkt 53 voneinander weg, wodurch ein Auseinanderreißen der Schläuche im erhitzten Trennbereich hervorgerufen wird (vgl. Figur 2F). Gleichzeitig oder nach der Bewegung der Scheiben 20a, 20b um den Drehpunkt 53 werden die Scheiben 20a, 20b in sich um ihre durch ihren jeweiligen Mittelpunkt verlaufenden Rotationsachsen gedreht, so dass sich die durch das Auseinanderreißen der Schläuche 1a, 1 b entstandenen Enden der Hauptschlauchabschnitte 100a, 100b annähern; vergleiche Figur 3E.

Nach Rotation der Scheiben 20a, 20b um ihren jeweiligen Mittelpunkt (um etwa 90°) sind die Scheiben 20a, 20b und damit auch die zu verbindenden Enden der Hauptschlauchabschnitte 100a, 100b aufgrund der durch die Mittel 50 erzeugten Rotation um den Drehpunkt 53 noch voneinander beabstandet. Erst durch weitere Drehung der Ringscheibe 55 werden die unteren Enden 514, 515 der Arme 51, 52 wieder einander angenähert, so dass sich die Kreisscheiben 20a, 20b aufeinander zu bewegen und die Enden der Hauptschlauchabschnitte 100a, 100b miteinander in Kontakt kommen; vergleiche Figur 3F.

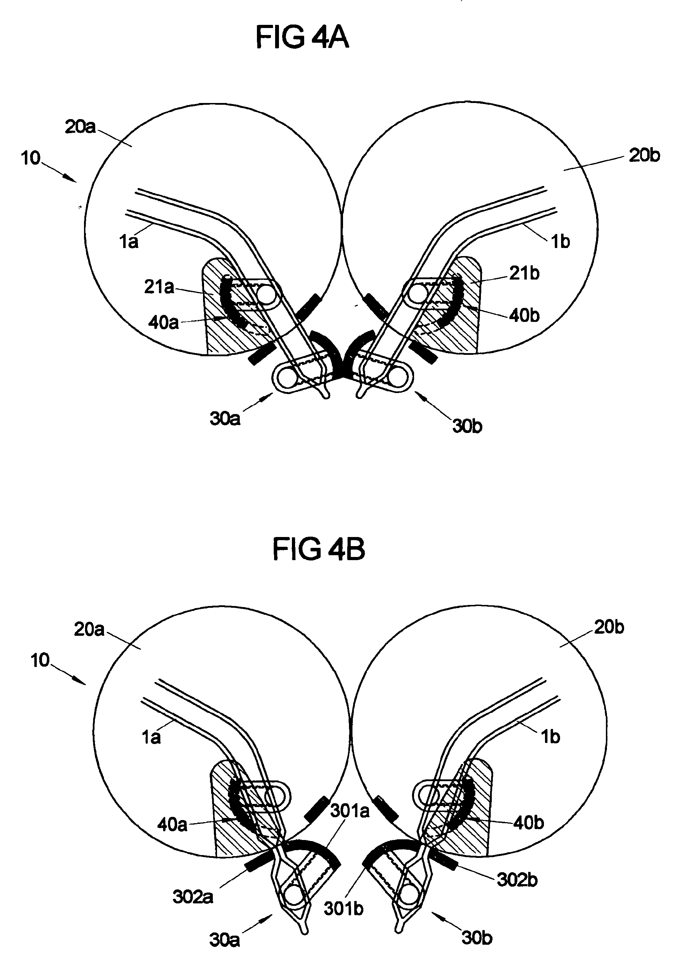

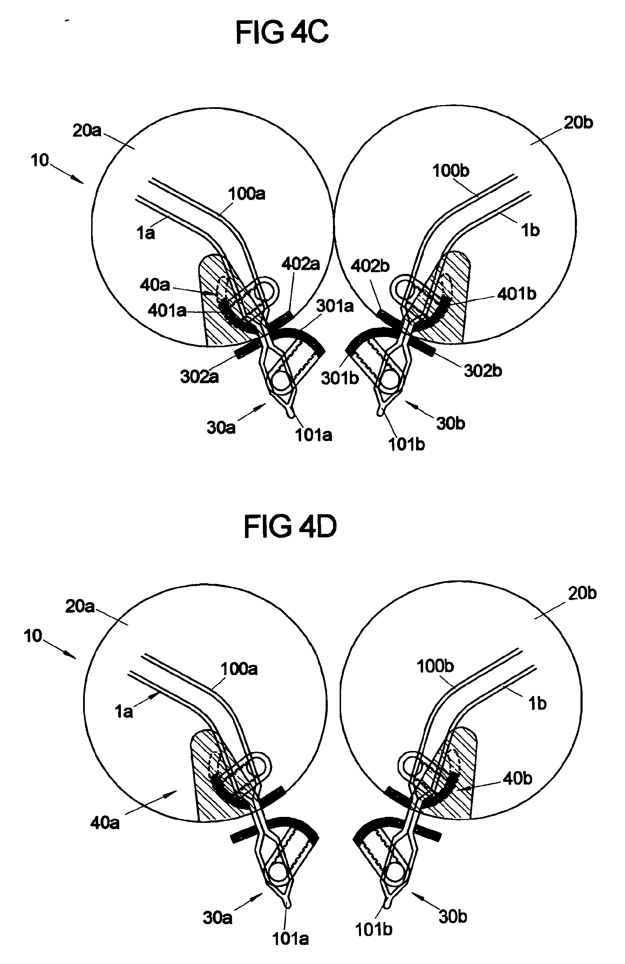

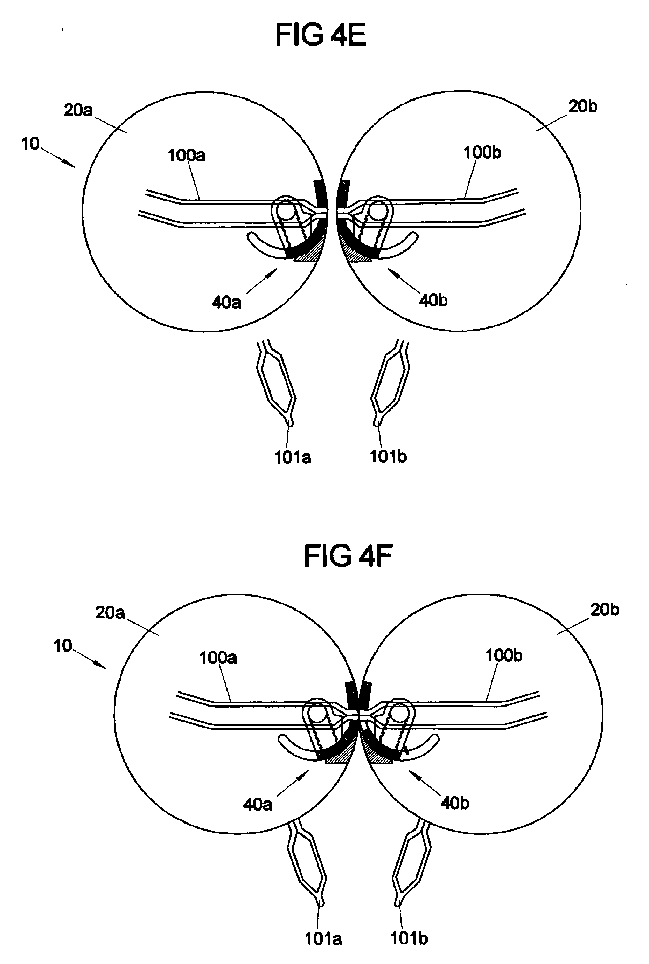

Die Figuren 4A bis 4G betreffen eine Vorrichtung gemäß einem weiteren Ausführungsbeispiel der Erfindung. Die Vorrichtung 10 dieses Ausführungsbeispiels entspricht im Prinzip dem Aufbau der Vorrichtung der Figuren 2A bis 2D. Allerdings sind die Aufnahmekörper 40a, 40b etwas anders gestaltet und insbesondere so angeordnet, dass sie in Ausgangsposition (Figur 4A) der Scheiben 20a, 20b so orientiert sind, dass sich ihre Seiten, in denen jeweils die Nut zur Aufnahme des Schlauches 1a, 1 b vorgesehen ist, voneinander weg erstrecken.

Insbesondere sind die Aufnahmeseiten der Aufnahmekörper 40a, 40b so schräg zu einer Vertikalen (z.B. einer Symmetrieebene der Vorrichtung) ausgerichtet, dass die in ihre Nuten eingelegten Schläuche 1 a, 1 b aufeinander zu laufen, das heißt Schlauchabschnitte in der Nähe der ersten Klemmvorrichtung 30a, 30b haben in Ausgangsposition der Vorrichtung 10 einen geringen Abstand zueinander als Schlauchabschnitte, die sich in einem größeren Abstand von der ersten Klemmvorrichtung befinden. Diese Orientierung der Aufnahmekörper 21 a, 21 b ermöglicht insbesondere ein erleichtertes Einlegen der Schläuche in die Vorrichtung 10.

Des Weiteren sind die Klemmbacken 301 a, 302a bzw. 301 b, 302b der ersten Klemmvorrichtungen 30a, 30b im Vergleich zum Ausführungsbeispiel der Figuren 2A bis 2H gespiegelt angeordnet, d.h. die erste Klemmbacke 301 a der Klemmvorrichtung 30a wird gegen den Uhrzeigersinn gedreht, um sie in Klemmstellung zu bringen, die erste Klemmbacke 301 b der Klemmvorrichtung 30b im Uhrzeigersinn. Die Aufnahmekörper 21 a, 21 b und die ersten Klemmvorrichtungen 30a, 30b sind aus Gründen der Übersichtlichkeit in den Figuren 4E bis 4G nicht dargestellt.

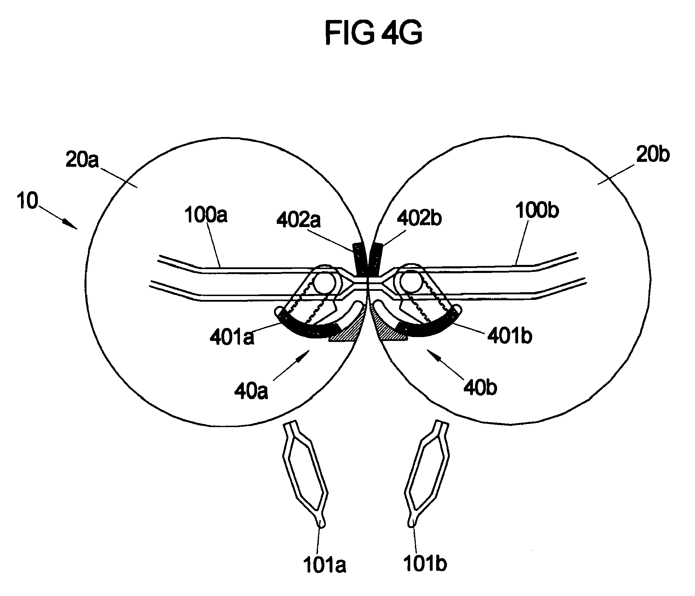

Figur 4G zeigt das in den Figuren 2A bis 2H nicht dargestellte Freigeben des Schlauches nach dem Verbinden der ersten Schlauchabschnitte, wobei die Klemmbacken 401 a, 401 b der zweiten Klemmvorrichtung insbesondere nach einer Abkühlzeit von dem Schlauch entfernt werden, so dass er aus der Vorrichtung entnommen werden kann.

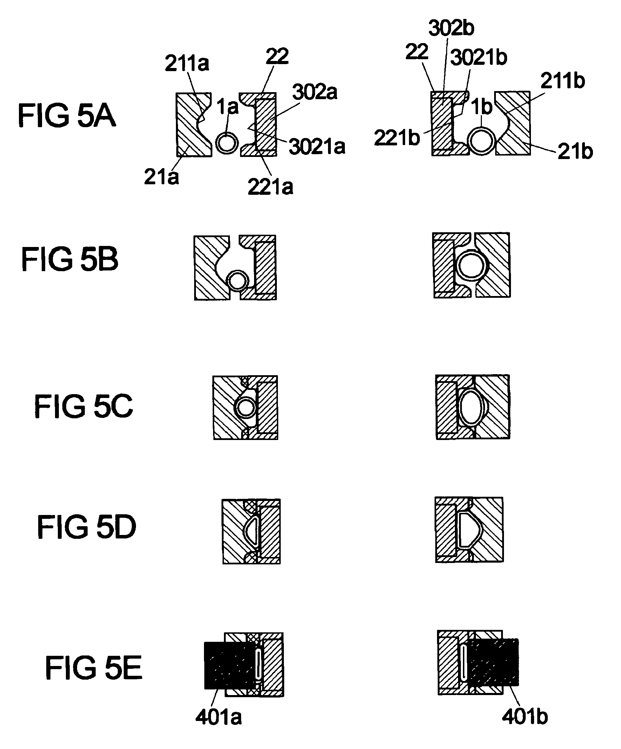

Die Figuren 5A bis 5E zeigen im Detail die durch die Form der Nuten 211 a, 211 b in den Aufnahmekörpern 40a, 40b und dem Führungskörper 22 bewirkte Zentrierung der Schläuche 1 a, 1 b, wobei der Schlauch 1 a einen kleineren Durchmesser aufweist als der Schlauch 1 b.

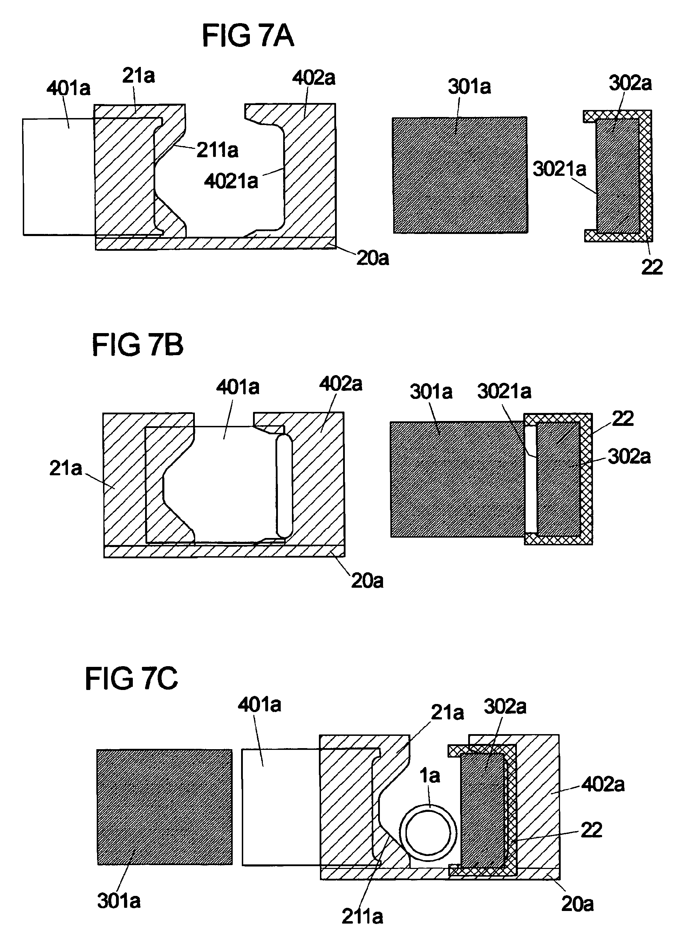

Durch die Zentrierung der beiden Schläuche wird sichergestellt, dass sich nach dem Auftrennen der Schläuche 1a, 1 b und dem Ausrichten der Enden der Hauptschlauchabschnitte 100a, 100b (vergleiche z.B. die oben diskutierte Figur 2G), die Mittelpunkte der Schlauchquerschnitte zumindest näherungsweise auf der gemeinsamen Längsachse der sich entlang dieser Längsachse erstreckenden Bereiche der Hauptschlauchabschnitte 100a, 100b befinden. Hierdurch können auch Schläuche unterschiedlichen Durchmessers mit möglichst guter Verbindungsqualität miteinander verbunden werden. Eine andere Ausgestaltung der Nuten der Aufnahmekörper 40a, 40b bzw. des Führungskörpers 22 ist in den Figuren 7A bis 7F gezeigt.

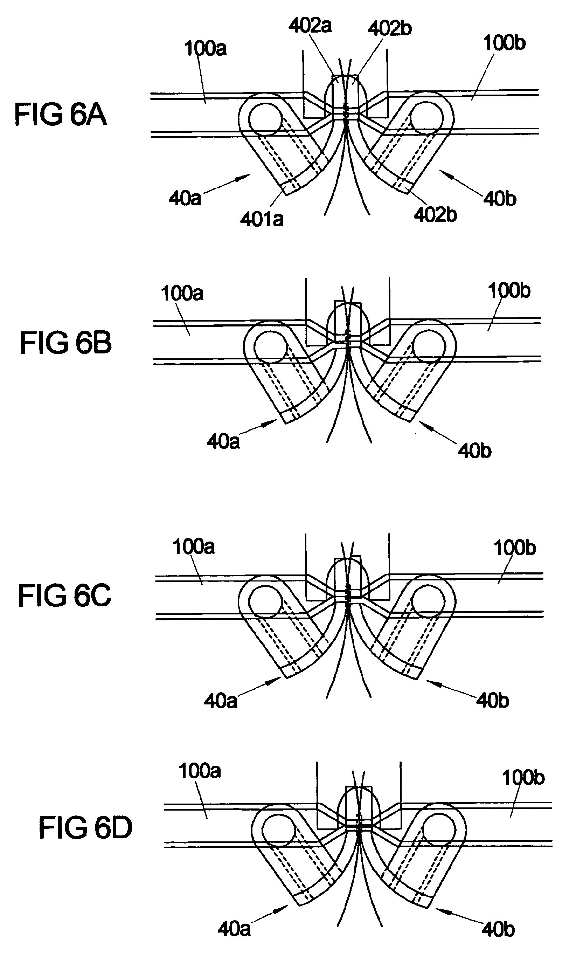

Die Figuren 6A bis 6D zeigen die Situation nach Herstellen des mechanischen Kontaktes (analog zu Figur 2H) zwischen den Enden der Hauptschlauchabschnitte 100a, 100b, wobei unterschiedliche Durchmesser der miteinander verbundenen Hauptschlauchabschnitte 100a, 100b betrachtet werden. Gemäß Figur 6A weisen die beiden Hauptschlauchabschnitte 100a, 100b den gleichen Durchmesser auf, so dass ihre Stirnseiten nach dem Herstellen des mechanischen Kontaktes nahezu vollständig aufeinander liegen. Figur 6D betrachtet einen ähnlichen Fall, wobei die beiden Hauptschlauchabschnitte 100a, 100b jedoch eine größere Wandstärke aufweisen.

Gemäß Figur 6B weist der eine Schlauchabschnitt 100a einen größeren Durchmesser auf als der andere Hauptschlauchabschnitt 100b, in Figur 6C ist es umgekehrt. In beiden Fällen entsteht jedoch durch den anhand der Figuren 5A bis 5E illustrierten Zentriermechanismus kein Versatz zwischen den Hauptschlauchabschnittsenden, so dass auch Schläuche unterschiedlichen Durchmessers sicher miteinander verbunden werden können.

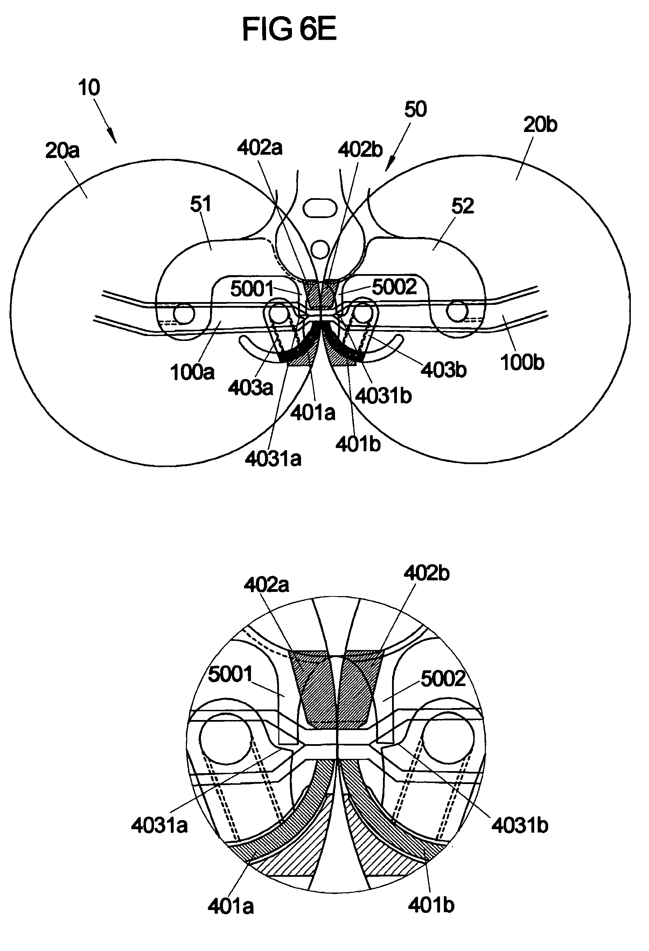

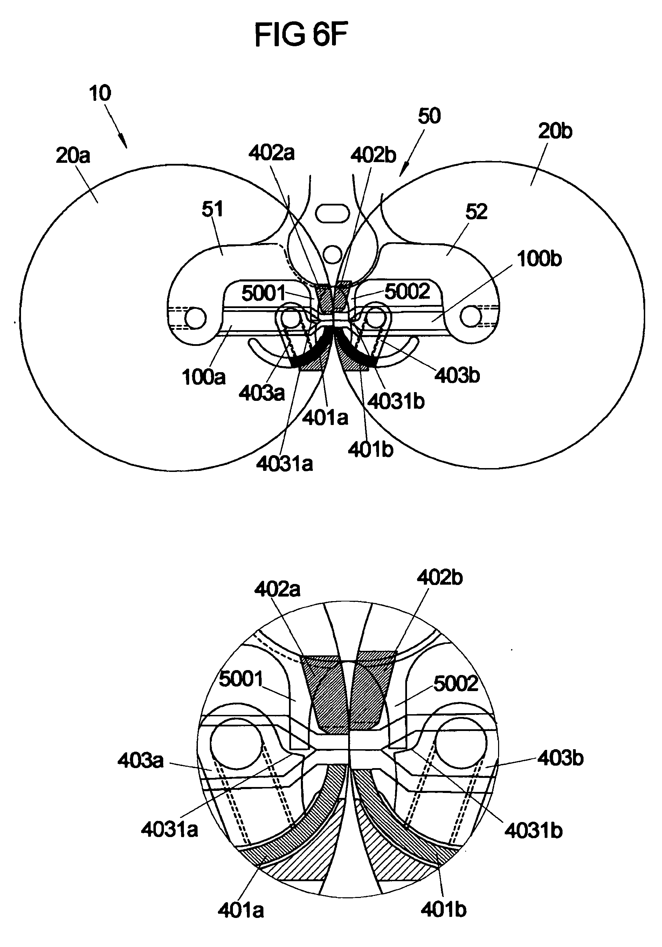

Die Figuren 6E und 6F beziehen sich auf eine Möglichkeit, unterschiedlich dicke erste Schlauchabschnitte möglichst reproduzierbar durch Rotation der Scheiben 20a, 20b in eine Position zu bringen, in der sich ihre Enden entlang einer gemeinsamen Längsachse gegenüber liegen. Danach weist die Vorrichtung 10 analog zu den Figuren 3A bis 3F Bewegungserzeugungsmittel 50 in Form einer Schere auf, von der die Figuren 6E und 6F lediglich einen unteren Bereich zeigen, d.h. insbesondere einen unteren Abschnitt der Arme 51,52.

Die Arme 51, 52 der Bewegungserzeugungsmittel 50 bilden jeweils einen Anschlag in Form eines Vorsprungs 5001, 5002 aus, die mit Armen 403a, 403b zusammenwirken, über die die Klemmbacken 401a, 401b rotiert werden können und die auf derselben Seite der Scheiben 20a, 20b wie die Arme 51, 52 angeordnet sind.

Die Arme 403a, 403b sind über eine Aussparung in der jeweiligen Scheibe 20a, 20b mit den Klemmbacken 401 a, 401 b verbunden.

Die Arme 403a, 403b bilden jeweils eine Anlagefläche 4031 a, 4031 b aus, wobei die Scheiben 20a, 20b, um die Schlauchabschnitte 100a, 100b entlang einer gemeinsamen Längsachse auszurichten, soweit gedreht werden, bis die Anlageflächen 4031 a, 4031 b an den Vorsprüngen 5001, 5002 zur Anlage kommen. Mittels der Vorsprünge 5001, 5002 wird somit ein definiertes Rotieren der Scheiben 20a, 20b ermöglicht, derart, dass die Schlauchabschnitte unabhängig von ihrem Durchmesser jeweils in eine Position gebracht werden, in der die Achsen ihrer Enden fluchten; vgl. insbesondere die unterhalb der Hauptdarstellung gezeigte Ausschnittsvergrößerung der Fig. 6F, wonach zwei Schlauchabschnitte mit unterschiedlichen Durchmessern so positioniert werden, dass ihre Mittelachse fluchten. Fig. 6E bezieht sich auf den Fall, dass die beiden Schlauchabschnitte denselben Durchmesser aufweisen.

Die Figuren 7A bis 7F beziehen sich auch eine Ausgestaltung von Klemmvorrichtungen, mit denen die Schläuche vor dem Auftrennen zentriert werden können. Das Prinzip des Zentrierens wurde bereits anhand der Fig. 5A bis 5E erläutert. Derartige Klemmvorrichtungen können z.B. in dem Ausführungsbeispiel der Figuren 2A bis 2H realisiert sein.

Die Figuren 7A und 7B zeigen die grundsätzliche Ausgestaltung der Klemmvorrichtungen, während die Figuren 7C bis 7F Schritte beim Klemmen eines Schlauches mittels der Klemmvorrichtungen darstellen.

Gemäß Fig. 7A ist analog zu Fig. 2A eine erste Klemmvorrichtung mit einer ersten rotierbaren Klemmbacke 301 a und einer zweiten Klemmbacke 302a (die gleichzeitig eine "heiße" HF-Elektrode ist, wie oben beschrieben), die in einem (insbesondere isolierendem) Führungskörper 22 angeordnet ist, vorgesehen. Des Weiteren ist ein Aufnahmekörper 21 a mit einer Nut 211 a zur Führung eines Schlauches sowie eine zweite Klemmvorrichtung an einer Aufnahmevorrichtung (Scheibe 20a) festgelegt, wobei die zweite Klemmvorrichtung eine erste rotierbare Klemmbacke 401 a sowie eine zusammen mit dem Aufnahmekörper 21 a auf der Scheibe 20a angeordnete zweite Klemmbacke 402a umfasst.

Fig. 7A zeigt die Klemmvorrichtungen in Ausgangsposition, Fig. 7B nach Rotieren der Klemmbacken 301 a und 401 a. Die in den Fig. 7A bis 7F gezeigten Klemmvorrichtungen entsprechen insbesondere der linken Seite der Vorrichtung 10 aus Fig. 2A, d.h. sie sind jeweils der Scheibe 20a zugeordnet. Die Klemmvorrichtungen der rechten Seiten in Fig. 2A (d.h. der zweiten Scheibe 20b der Vorrichtung 10) können jedoch analoge Klemmvorrichtungen aufweisen.

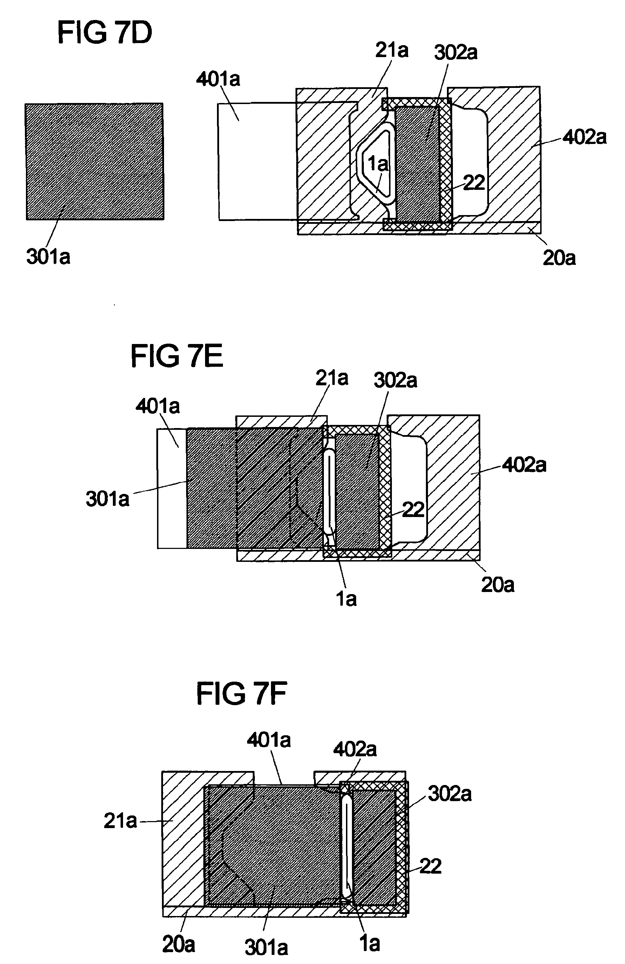

Zum Klemmen eines Schlauches 1a wird dieser in Ausgangsstellung der Klemmvorrichtungen (Fig. 7C, analog zu Fig. 2A) insbesondere zwischen der Nut 211a des Aufnahmekörpers 21 a und der Klemmbacke 402a positioniert. Anschließend erfolgt eine Rotation der Scheibe 20a, so dass der Schlauch 1 a gegen die Nut 211 a und die Klemmseite 3021 a der Klemmbacke 302a gedrückt wird; vgl. Fig. 7D (entsprechend Fig. 2B). Durch die V-förmige Gestalt der Nut 211 a und einer ebenfalls U- oder V-förmig gestalteten Klemmseite 4021 a der Klemmbacke 402a wird der Schlauch 1 a zentriert.

Nun erfolgt eine Rotation der Klemmbacke 301 a, so dass der Schlauch 1 a zwischen den Klemmbacken 301 a und 302a zusammengedrückt wird, wie in Fig. 7E dargestellt (vgl. Fig. 2C). Anschließend erfolgt ein Zurückrotieren der Scheibe 20a in Richtung ihrer Ausgangsposition und ein Verschwenken der Klemmbacke 401 a, so dass der Schlauch 1a sowohl zwischen den Klemmbacken 301 a und 302a als auch zwischen den Klemmbacken 401 a, 402a geklemmt ist (Fig. 7F, entsprechend Fig. 2D).

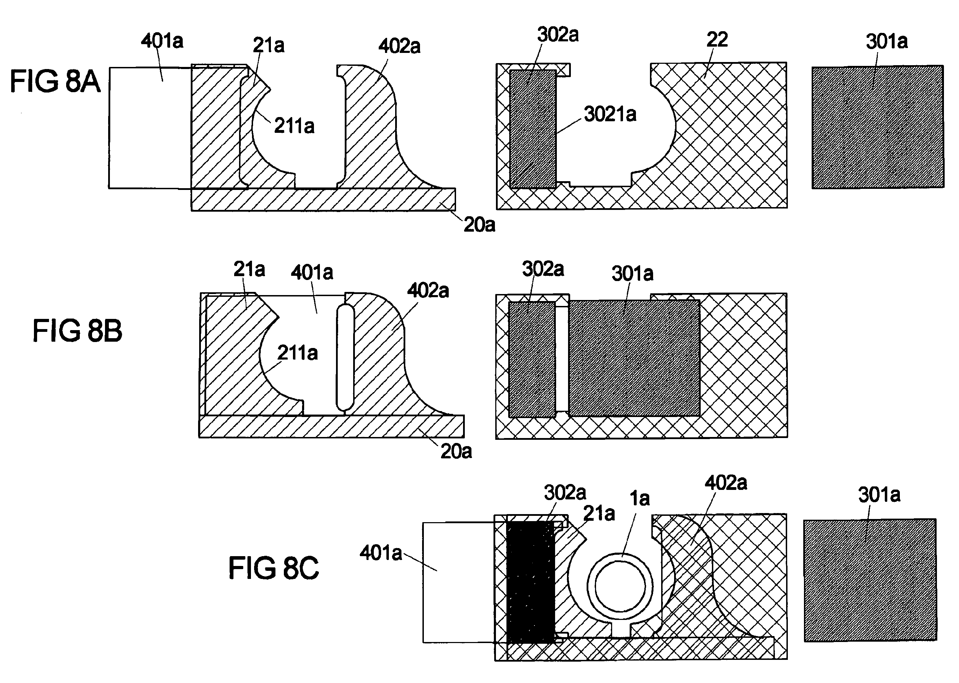

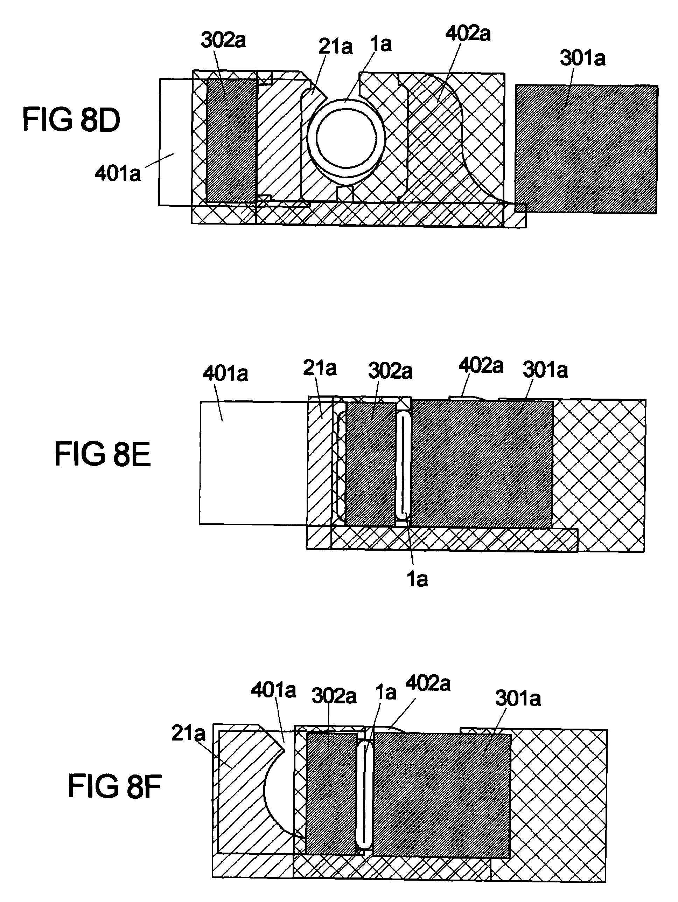

Die Figuren 8A bis 8F zeigen eine Abwandlung der in den Figuren 7A bis 7F dargestellten Klemmvorrichtungen, die insbesondere in einer Vorrichtung gemäß dem Ausführungsbeispiel der Figuren 4A bis 4G realisiert sein können.

Analog zu den Figuren 7A und 7B zeigen die Figuren 8A und 8B die grundsätzliche Ausgestaltung der Klemmvorrichtungen, während die Figuren 8C bis 8F Schritte beim Klemmen eines Schlauches mittels der Klemmvorrichtungen darstellen. Wie die Figuren 7A bis 7F beziehen sich die Figuren 8A bis 8F auf Klemmvorrichtungen, die einer Scheibe 20a der Vorrichtung zugeordnet sind. Die Klemmvorrichtungen der anderen Scheibe 20b können jedoch analog ausgebildet sein.

Im Unterschied zu den Figuren 7A bis 7F sind die Klemmbacken 301 a und 302a "vertauscht", d.h. sie befinden sich auf der jeweils anderen Seite des zu klemmenden Schlauches, weshalb der Führungskörper 22 anders gestaltet ist und z.B. den Schlauch an einer Seite umgreift; vgl. Fig. 8A. Des Weiteren ist die Nut 211 a in dem Aufnahmekörper 21 a etwas anders gestaltet, nämlich mit einem zumindest näherungsweise teilkreisartigen Querschnitt. Gleichzeitig verläuft die Klemmseite 4021 a der Klemmbacke 402a flacher, z.B. zumindest auf Höhe der Nut 211 a eben. Die in den Figuren 8C bis 8F dargestellten Positionen der Klemmbacken und der Scheibe 20a entsprechen den Positionen der Klemmbacken in den Figuren 7C bis 7F.

Es wird darauf hingewiesen, dass Elemente der obigen Figuren natürlich im Prinzip in beliebiger Kombination miteinander verwendet werden können. Beispielsweise können die Bewegungserzeugungsmittel der Figuren 3A bis 3F zusammen mit der Ausführung der Vorrichtung gemäß den Figuren 4A bis 4G verwendet werden. Es ist z.B. auch denkbar, dass unterschiedlich gestaltete Klemmbacken verwendet werden; z.B. werden der ersten Scheibe 20a Klemmbacken gemäß den Fig. 7A bis 7F zugeordnet und der zweiten Scheibe Klemmbacken gemäß den Fig. 8A bis 8F.

### Bezugszeichenliste

- 1a, 1b: Schlauch
- 20a, 20b: rotierbare Scheibe
- 21 a, 21 b: Aufnahmekörper
- 22: Führungskörper
- 30a, 30b: erste Klemmvorrichtung
- 40a, 40b: zweite Klemmvorrichtung
- 50: Bewegungserzeugungsmittel
- 51: erster Arm
- 52: zweiter Arm
- 53: Drehpunkt
- 55: Ringscheibe
- 56, 57: Verbindungselemente
- 100a, 100b: erster Schlauchabschnitt
- 101 a, 101 b: zweiter Schlauchabschnitt
- 211a, 211b: Nut
- 221 a, 221 b: Nut
- 301 a, 301 b: erste Klemmbacke erste Klemmvorrichtung
- 302a, 302b: zweite Klemmbacke erste Klemmvorrichtung
- 303a,303b,403a,403b: Arm
- 305a: Klemmfläche erste Klemmbacke
- 306a: Klemmfläche zweite Klemmbacke
- 401 a, 401 b: erste Klemmbacke zweite Klemmvorrichtung
- 402a, 402b: zweite Klemmbacke zweite Klemmvorrichtung
- 403a,403b: Arm
- 511, 521: Verbindungsmittel
- 512, 513: oberes Ende
- 514,515: unteres Ende
- 1001 a, 1001 b: Ende erster Schlauchabschnitt
- 3021 a, 3021 b: Klemmseite
- 4021 a: Klemmseite
- 4031 a, 4031 b: Anlagefläche
- 5001, 5002: Vorsprung

## Patentansprüche

1. Verfahren zum sterilen Verbinden von Schläuchen, mit den Schritten:
a) Bereitstellen eines ersten und eines zweiten Schlauchs (1a, 1 b);
b) Erhitzen jeweils eines Trennbereiches des ersten und des zweiten Schlauchs (1 a, 1 b) unter Verwendung von Erhitzungsmitteln;
c) mechanisches Auftrennen der beiden Schläuche (1 a, 1 b) durch Ausüben einer Zug- und/oder Scherkraft auf die Schläuche, so dass die beiden Schläuche (1 a, 1 b) jeweils im erhitzten Trennbereich in einen ersten und einen zweiten Schlauchabschnitt (100a, 100b, 101 a, 101 b) aufgetrennt werden; und
d) nach dem Auftrennen der Schläuche (1a, 1 b) Herstellen eines mechanischen Kontaktes des durch das Auftrennen des ersten Schlauchs gebildeten Endes (1001 a) des ersten Schlauchabschnitts (100a) des ersten Schlauchs (1 a) mit dem durch das Auftrennen des zweiten Schlauchs (1 b) gebildeten Ende (1001 b) des ersten Schlauchabschnitts (100b) des zweiten Schlauchs (1 b), wobei
e) das Herstellen des mechanischen Kontaktes so erfolgt, dass die Enden (1001 a, 1001 b) der Schlauchabschnitte (100a, 100b) in Folge des Erhitzens gemäß Schritt b) noch eine solche Temperatur aufweisen, wenn sie in mechanischen Kontakt miteinander kommen, dass sie ohne zusätzliches Erhitzen nach dem Auftrennen der Schläuche eine stoffschlüssige Verbindung miteinander eingehen.

2. Verfahren nach Anspruch 1, **dadurch gekennzeichnet, dass** die Zug- und/oder Scherkraft zum Auftrennen der Schläuche (1 a, 1 b) durch eine Rotation und/oder eine Translation eines Teiles des jeweiligen Schlauches (1 a, 1 b) erzeugt wird.

3. Verfahren nach Anspruch 1 oder 2, **dadurch gekennzeichnet, dass** das Herstellen des mechanischen Kontaktes so erfolgt, dass die Enden (1001 a, 1001 b), wenn sie in mechanischen Kontakt miteinander kommen, jeweils eine Temperatur aufweisen, die größer als die Schmelztemperatur des Materials des jeweiligen Schlauchabschnittes (100a, 100b) ist.

4. Verfahren nach einem der vorhergehenden Ansprüche, **dadurch gekennzeichnet, dass** der zeitliche Abstand zwischen dem Zeitpunkt des Vorliegens des mechanischen Kontaktes und dem Auftrennen des ersten und des zweiten Schlauches (1 a, 1 b) höchstens 0,1 s, insbesondere höchstens 0,05 s, beträgt.

5. Verfahren nach einem der vorhergehenden Ansprüche, **dadurch gekennzeichnet, dass** das Herstellen des mechanischen Kontaktes zwischen den Enden (1001 a, 1001 b) der ersten Schlauchabschnitte (100a, 100b) eine Rotation zumindest eines Teils des ersten Schlauchabschnitts (100a) des ersten Schlauches (1a) und/oder eine Rotation zumindest eines Teils des ersten Schlauchabschnitts (100b) des zweiten Schlauches (1b) umfasst.

6. Verfahren nach Anspruch 5, **dadurch gekennzeichnet, dass** die Rotation des ersten Schlauchabschnitts (100a) des ersten Schlauches (1a) und/oder des ersten Schlauchabschnitts (100b) des zweiten Schlauches (1 b) um eine Rotationsachse erfolgt, die zumindest näherungsweise senkrecht zu einer Ebene verläuft, entlang derer sich der erste Schlauchabschnitt (100a, 100b) zumindest teilweise erstreckt.

7. Vorrichtung zum sterilen Verbinden von Schläuchen, insbesondere zum Durchführen eines Verfahrens gemäß einem der vorhergehenden Ansprüche, mit
- einer ersten Aufnahmevorrichtung (20a) zur Aufnahme eines ersten Schlauches (1a);
- einer zweiten Aufnahmevorrichtung (20b) zur Aufnahme eines zweiten Schlauches (1 b);
- Erhitzungsmitteln zum Erhitzen eines Trennbereiches eines an der ersten Aufnahmevorrichtung (20a) angeordneten ersten Schlauchs (1a) und eines Trennbereichs eines an der zweiten Aufnahmevorrichtung (20b) angeordneten zweiten Schlauchs (1b); und
- Auftrennmitteln zum Auftrennen des ersten und des zweiten Schlauchs (1a, 1 b) in dem erhitzten Trennbereich jeweils in einen ersten und einen zweiten Schlauchabschnitt (1 00a, 100b, 101 a, 101 b), wobei
- die erste und die zweite Aufnahmevorrichtung (20a, 20b) jeweils ausgebildet sind, den ersten Schlauchabschnitt (100a, 100b) jeweils so zu halten, dass er sich zumindest teilweise entlang einer Ebene erstreckt, und zumindest eine der beiden Aufnahmenvorrichtungen (20a, 20b) um eine Rotationsachse rotierbar gelagert ist, die zumindest näherungsweise senkrecht zu der Ebene verläuft, und wobei
- die Vorrichtung (10) so ausgebildet ist, dass nach Auftrennen des ersten und des zweiten Schlauches (1 a, 1 b) ein Ende (1001 a) des ersten Schlauchabschnitts (100a) des ersten Schlauches (1 a) unter Rotation der ersten und/oder der zweiten Aufnahmevorrichtung (20a, 20b) um die Rotationsachse in mechanischen Kontakt mit einem Ende (1001 b) des ersten Schlauchabschnitts (100b) des zweiten Schlauches (1 b) gebracht werden kann.

8. Vorrichtung nach Anspruch 7, **dadurch gekennzeichnet, dass** jeder Aufnahmevorrichtung (20a, 20b) mindestens eine Klemmvorrichtung (30a, 30b) zum Klemmen eines an der Aufnahmevorrichtung angeordneten Schlauchs (1a, 1 b) zugeordnet ist.

9. Vorrichtung nach Anspruch 8, **dadurch gekennzeichnet, dass** die Erhitzungsmittel Hochfrequenzspannungserzeugungsmittel umfassen, die jeweils mit zumindest einer der Klemmvorrichtungen (30a, 30b) elektrisch verbunden und ausgebildet sind, eine Hochfrequenzspannung in die jeweilige Klemmvorrichtung (30a, 30b) einzuleiten, um im Bereich der Klemmvorrichtung (30a, 30b) ein elektrisches Hochfrequenzfeld zu erzeugen, das den Trennbereich des mittels der Klemmvorrichtung (30a, 30b) geklemmten Schlauchs (1a, 1 b) erhitzt.

10. Vorrichtung nach Anspruch 8 oder 9, **dadurch gekennzeichnet, dass** jeder Aufnahmevorrichtung (20a, 20b) mindestens eine weitere Klemmvorrichtung (40a, 40b) zum Klemmen eines an der Aufnahmevorrichtung (20a, 20b) angeordneten Schlauchs (1a, 1 b) zugeordnet ist, die so an der jeweiligen Aufnahmevorrichtung (20a, 20b) angeordnet ist, dass sie bei Rotation der Aufnahmevorrichtung mitrotiert.

11. Vorrichtung nach einem der Ansprüche 7 bis 10, **dadurch gekennzeichnet, dass** die Auftrennmittel Bewegungserzeugungsmittel (50) zum Erzeugen einer Bewegung der ersten und/oder der zweiten Aufnahmevorrichtung (20a, 20b) umfassen, wobei durch eine Bewegung der ersten und/oder der zweiten Aufnahmevorrichtung (20a, 20b) eine Zug- und/oder Scherkraft auf den ersten und/oder den zweiten Schlauch ausgeübt wird, so dass er im erhitzten Trennbereich in den ersten und den zweiten Schlauchabschnitt aufgetrennt wird.

12. Vorrichtung nach Anspruch 11, **dadurch gekennzeichnet, dass** die Bewegungserzeugungsmittel (50) einen mit der ersten Aufnahmevorrichtung (20a) verbundenen ersten Arm (51) und einen mit der zweiten Aufnahmevorrichtung (20b) verbundenen zweiten Arm (52) umfassen, wobei die beiden Arme (51, 52) so miteinander gekoppelt sind, dass sie nach Art einer Schere um eine gemeinsame Rotationsachse (53) relativ zueinander rotierbar sind, so dass bei einer Rotation der Arme (51, 52) eine Rotation der Aufnahmevorrichtungen (20a, 20b) ebenfalls um diese Rotationsachse (53) erzeugt wird.

13. Vorrichtung nach einem der Ansprüche 11 oder 12, **dadurch gekennzeichnet, dass** die Bewegungserzeugungsmittel (50) zum Erzeugen einer Translation der ersten und/oder der zweiten Aufnahmevorrichtung (20a, 20b) ausgebildet sind.

14. Vorrichtung nach einem der Ansprüche 7 bis 13, **dadurch gekennzeichnet, dass** die erste und/oder die zweite Aufnahmevorrichtung (20a, 20b) jeweils plattenartig, insbesondere in Form einer Scheibe, ausgebildet sind.

15. Vorrichtung nach einem der Ansprüche 7 bis 14, **dadurch gekennzeichnet, dass** die erste und/oder die zweite Aufnahmevorrichtung (20a, 20b) jeweils einen Aufnahmekörper (21 a, 21 b) aufweisen, der an einer parallel zur Rotationsachse orientierten Seite eine Aussparung (211 a, 211 b) zum Einlegen des ersten oder des zweiten Schlauches (1a, 1b) aufweist.
